# EUROPEAN PATENT SPECIFICATION

(11) **EP 2 173 747 B1**
(45) Date of publication and mention of the grant of the patent: **21.12.2011**
(21) Application number: 08759157.4
(22) Date of filing: 11.06.2008
(51) Int. Cl.: C07D 471/04, C07D 471/14, C07D 487/04, C07D 491/14

(54) **A REGIOSELECTIVE METAL CATALYZED SYNTHESIS OF ANNELATED BENZIMIDAZOLES AND AZABENZIMIDAZOLES**
REGIOSELEKTIVE, METALLKATALYSIERTE SYNTHESE ANNELIERTER BENZIMIDAZOLE UND AZABENZIMIDAZOLE
SYNTHÈSE RÉGIOSÉLECTIVE CATALYSÉE PAR UN MÉTAL DE BENZIMIDAZOLES ET D'AZABENZIMIDAZOLES CONDENSÉS

(30) Priority: 26.06.2007 EP 07290801
(43) Date of publication of application: 14.04.2010
(73) Proprietor: SANOFI, 75013 Paris (FR)
(72) Inventor: ALONSO, Jorge, 68163 Mannheim (DE); LINDENSCHMIDT, Andreas, 65926 Frankfurt am Main (DE); NAZARE, Marc, 65926 Frankfurt am Main (DE); R'KYEN, Omar, Tangier (MA); URMANN, Mathias, 65926 Frankfurt am Main (DE); HALLAND, Nis, 65926 Frankfurt am Main (DE)
(74) Representative: Then, Johann
(86) International application number: PCT/EP2008/004638
(87) International publication number: WO 2009/000412

(56) References cited:
- WO-A-2006/058064
- WEI DENG ET. AL.: "Amino acid mediated Goldberg reactions between amides and aryl iodides." TETRAHEDRON LETTERS, vol. 45, 2004, pages 2311-2315, XP002494869 cited in the application
- R. G. BROWNING ET. AL.: "Palladion catalyzed aryl-amidation. Synthesis of non-racemic N-aryl lactams" TETRAHEDRON, vol. 60, 2004, pages 359-365, XP002494870 cited in the application
- DATABASE WPI Week 199128 1991, Thomson Scientific, London, GB; AN 1991-205956 XP002494871 "Dihydro-pyrrolo benzimidazole sulphonamide production used as plant growth promoters and intemediates in production of pesticides and medicines." & SU 1 587 052 A (PLANTS CHEM PROTECT) 1988

## Description

### Field of the Invention

The present invention relates to a process for the regioselective synthesis of compounds of the formula (I), 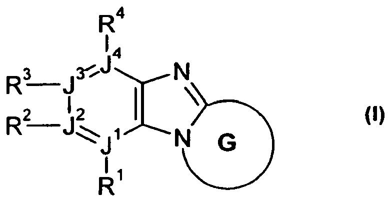 wherein R1; R2; R3; R4; J1; J2; J3; J4 and G have the meanings indicated below and which are useful as intermediates for the preparation of valuable pharmaceutically active ingredients.

### Background of the Invention

The present invention relates to a direct metal catalyzed, regioselective process for the preparation of a wide variety of unsymmetrical, multifunctional annelated benzimidazoles or azabenzimidazoles of the formula (I) starting from 2-halo-nitroarenes and lactames. Preferred metals are palladium and copper.

Annelated benzimidazoles play an important role in drug discovery and can certainly be regarded as privileged structures in pharmaceutical research. Several benzimidazole derivatives containing fused ring structures have anti-inflammatory, analgesic, antiarthritic, antitumor activity, or a combination of this activities (A. J. Charlson, J. S. Harrington, Carbohydrate Research 1975, 43, 383-387; P. Bender U.S. Patent 4186205, 1980; Chem. Abstr. 1980, 92, 181195; H. G. Alpermann Arzneim.-Forsch. 1966, 16, 1641; R. Zhou, E. B. Skibo J. Med. Chem. 1996, 39, 4321-4331).

In contrast to the great importance of this scaffold no general regioselective route to annelated benzimidazoles und annelated benzimidazoles has been described yet. The few methods available so far are multi-step processes often requiring harsh reaction conditions and are restricted in the substrate range, have poor cost-effectiveness and are thus of limited use (J. R. McLure, J. H. Custer, H. D. Schwarz, D. A. Lill, Synlett, 710-712; E. B. Skibo, I. Islam, W. G. Schulz, R. Zhou, L. Bess, R. Boruah Synlett, 1996, 297-309; F. Aldabbagh, W. R. Bowman Tetrahedron 1999, 55, 4109-4122). Although palladium-catalyzed protocols for the cross-coupling between aryl halides and lactames have been reported (J. Yin, S. L. Buchwald Org. Lett. 2000, 2, 1101-1104; D. J. Madar, H. Kopecka, D. Pireh, J. Pease, M. Pliushchev, R. J. Sciotti, P. E. Wiedeman, S. W. Djuric Tetrahedron Lett. 2001, 42, 3681-3684), only one example employing a 2-halo-nitroarene derivative has been reported. R. G. Browning, V. Badarinarayana, H. Mahmud, C. J. Lovely, describe in this example the coupling of 1-bromo-2-nitro-benzene and a pyrrolidin-2-one derivative in moderate yield *(*Tetrahedron 2004, 60, 359-365).

Although copper-catalyzed protocols for the cross-coupling between aryl halides and lactames have been reported, very few examples employing 2-halo-nitroarenes exist. Wei Deng, Ye-Feng Wang, Yan Zou, Lei Liu, Qing-Xiang Guo describe the coupling of 1-iodo-2-nitrobenzene with pyrrolidine-2-one *(*Tetrahedron Lett. 2004, 45, 2311-2315). WO2006/058064 describes the preparation of a 1,2,3,4-tetrahydropyrido[1,2-a] benzimidazole derivative obtained from the preparation of 4-bromo-3-nitro-benzoic acid methyl ester with tert-butyl-[6-oxo-4-(2,4,5-trifluorophenyl)piperidin-3-yl]-carbamate in the presence of copper(I)iodide as catalyst, potassium carbonate and N,N'-dimethyl-ethylenediamine to give methyl 4-[5-(tert-butoxycarbonyl)amino]-2-oxo-4-(2,4,5-trifluorophenyl)piperidin-1-yl]-3-nitrobenzoate.

However, no general applicability for the palladium-catalyzed cross-coupling of 2-halo-nitroarenes, in particular 2-chloro-nitroarenes, and lactamas was shown, and in addition no use was made to for the regioselective synthesis of annelated benzimidazoles or azabenzimidazoles.

The limited regioselective access annelated benzimidazoles or azabenzimidazoles often prevents the optimization of a potential drug substance or substance with for example agricultural application and is accompanied by poor cost-effectiveness. Thus the present invention is useful in preparing intermediates or end products of biological active compounds in pharmaceutical and agricultural applications.

### Summary of the invention

The present invention provides a direct metal catalyzed, regioselective synthetic route to a wide variety of unsymmetrical, multifunctional annelated benzimidazoles or azabenzimidazoles of formula I starting from 2-halo-nitroarenes of formula II and lactames of formula III. Preferred metals are palladium and copper. Thus one aspect of the invention is an efficient and general palladium catalyzed coupling method for substituted 2-halo-nitroarenes (step 1) to intermediates of formula IV. In another aspect of the invention, an efficient process is provided for the subsequent reductive aminocyclisation (step 2) of intermediates of formula IV, which can be either performed with the crude reaction mixture of step 1 or optionally after simple filtration through a pad of celite by using a reducing reagent.

The advantages of the provided process are that it comprises a novel, direct regioselective catalytic, mild and general method for the synthesis of annelated benzimidazoles or azabenzimidazoles, which also can be performed as a one-pot procedure. Thus, the process is very time- and cost-effective. Moreover, are the reaction conditions compatible with a broad range of functional groups and a large variety of starting materials, which are easily accessible or even commercially available.

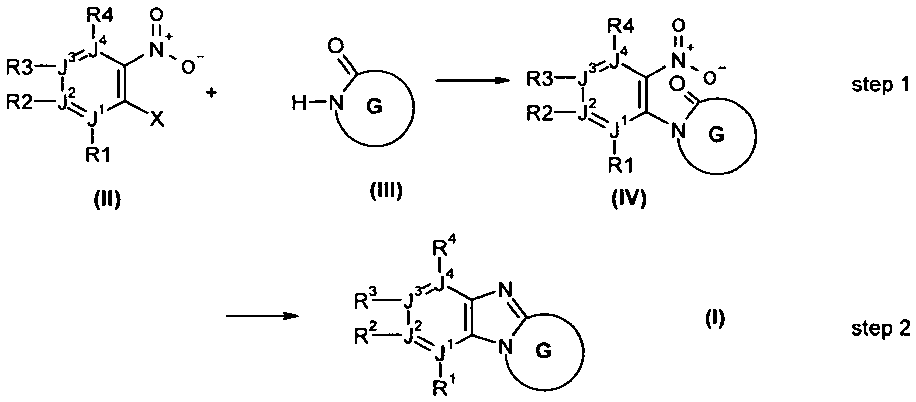

### Detailed description of the invention

A process for preparing a compound of formula I 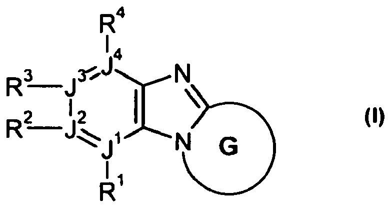 and/or all stereoisomeric forms of the compound of formula I, and/or mixtures of these forms in any ratio, and/or a physiologically tolerated salt of the compound of formula I, wherein
J1, J2, J3 and J4 are independently from each other selected from carbon or nitrogen atoms and form together with the carbon atoms they are attached to a stable aromatic or heteroaromatic ring,
G is selected from azetidine, azepane, azocane, aza-bicyclo[2.2.1]heptane, azabicyclo[2.2.2]octane, azacyclooctanone, azacyclononanone, aza-tricyclo [4.3.1.1*3,8*]undecane, 4,4-dimethyl-3,5-dioxa-azatricyclo[5.2.1.0*2,6*]-decane, 3,5-dioxa-azatricyclo-[5.2.1.0*2,6*]decane, 4,4-dimethyl-3,5-dioxa-azatricyclo [5.2.1.0*2,6*]decan-9-one, azocane-2-one, azonane, 1,4-diazepane, [1,4]diazocane, [1,2]diazocan-3-one, [1,3]diazocan-2-one, imidazoline, imidazolidine, isothiazolidine, isoxazolidine, ketopiperazine, morpholine, [1,4]oxazocane, [1,3]oxazocan-2-one, piperazine, piperidine, pyrazoline, pyrazolidine, 1,2-dihydro-pyridine, pyrrolidine, pyrrolidinone, 2,3-dihydro-1H-pyrrole, pyrroline, 5,6,7,8-tetrahydro-1H-azocin-2-one, tetrahydropyridine, thiadiazine, thiazolidine, thiazoline or thiomorpholine, wherein G is unsubstituted or mono-, di-, tri- or four times substituted independently of one another by oxo or by R5,
R1, R2, R3, R4 and R5 are independent of one another identical or different and are
a) hydrogen atom,
b) -(C₁-C₄)-alkyl, wherein alkyl is unsubstituted or substituted one to three times by R13,
c) halogen,
d) phenyloxy-, wherein phenyloxy is unsubstituted or substituted one to three times by R13,
e) -(C₁-C₃)-fluoroalkyl,
f) -N(R10)-(C₁-C₄)-alkyl, wherein alkyl is unsubstituted or substituted one to three times by R13,
g) -(C₆-C₁₄)-aryl, wherein aryl is unsubstituted or mono-, di-, tri- or four times substituted independently of one another by R13,
h) -(C₄-C₁₄)-heteroaryl, wherein heteroaryl is unsubstituted or mono-, di-, tri- or four times substituted independently of one another by R13,
i) -(C₃-C₈)-cycloalkyl, wherein said cycloalkyl is unsubstituted or mono-, di-, tri- or four times substituted independently of one another by R13,
j) a 3- to 7-membered cyclic residue, containing 1, 2, 3 or 4 heteroatoms chosen from nitrogen, sulfur or oxygen, wherein said cyclic residue is unsubstituted or mono-, di-, tri- or four times substituted independently of one another by R13,
k) -O-CF₃,
l) -O-(C₁-C₄)-alkyl, wherein alkyl is unsubstituted or substituted one to three times by R13,
m) -NO₂,
n) -CN,
o) -OH,
p) -C(O)-R10,
q) -C(O)-O-R 11,
r) -C(O)-N(R11)-R12,
s) -N(R11)-R12,
t) -N(R10)-SO₂-R10,
v) -S-R10,
w) -SOₙ-R10, wherein n is 1 or 2,
x) -SO₂-N(R11)-R12 or
y) at least one of R1, R2, R3 or R4 are absent in case one or more of J1, J2, J3 or J4 are nitrogen atom, or
R1 and R2, R2 and R3 or R3 and R4 form together with the atoms which they are attached to a 5- or 8- membered ring, containing up to 0, 1, 2, 3 or 4 heteroatoms chosen from nitrogen, sulfur or oxygen, wherein said ring is unsubstituted or substituted one, two, three or four times by R14,
R10 is hydrogen atom, -(C₁-C₃)-fluoroalkyl or -(C₁-C₆)-alkyl,
R11 and R12 are independently of one another identical or different and are
a) hydrogen atom,
b) -(C₁-C₆)-alkyl, wherein alkyl is unsubstituted or mono-, di- or trisubstituted independently of one another by R13,
c) -(C₆-C₁₄)-aryl-, wherein aryl is unsubstituted or mono-, di- or trisubstituted independently of one another by R13,
d) -(C₄-C₁₄)-heteroaryl, wherein heteroaryl is unsubstituted or mono-, di- or trisubstituted independently of one another by R13 or
R13 is halogen, -NO₂, -CN, =O, -OH, -(C₁-C₈)-alkyl, -(C₁-C₈)-alkoxy, -CF₃, phenyloxy-, -C(O)-R10, -C(O)-O-R17, -C(O)-N(R17)-R18, -N(R17)-R18, -N(R10)-SO₂-R10, -S-R10, -SOₙ-R10, wherein n is 1 or 2, -SO₂-N(R17)-R18, -(C₆-C₁₄)-aryl, wherein aryl is unsubstituted or mono-, di- or trisubstituted independently of one another by R14, -(C₄-C₁₄)-heteroaryl, wherein heteroaryl is unsubstituted or mono-, di- or trisubstituted independently of one another by R14, -(C₃-C₈)-cycloalkyl, wherein said cycloalkyl is unsubstituted or mono-, di- or trisubstituted independently of one another by R14, or a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14, R14 is halogen, -OH, =O, -CN, -CF₃, -(C₁-C₈)-alkyl, -(C₁-C₄)-alkoxy, -NO₂, -C(O)-OH, -NH₂, -C(O)-O-(C₁-C₄)-alkyl, -(C₁-C₈)-alkylsulfonyl, -C(O)-NH-(C₁-C₈)-alkyl, -C(O)-N-[(C₁-C₈)-alkyl]₂, -C(O)-NH₂, -S-R10, -N(R10)-C(O)-NH-(C₁-C₈)-alkyl, or -N(R10)-C(O)-N-[(C₁-C₈)-alkyl]₂,
R17 and R18 are independently of one another identical or different and are
a) hydrogen atom,
b) -(C₁-C₆)-alkyl,
c) -(C₆-C₁₄)-aryl- or
d) -(C₄-C₁₄)-heteroaryl,
   said process comprises a reaction of a compound of formula II 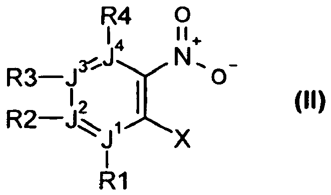
wherein R1, R2, R3, R4, J1, J2, J3 and J4 are as defined in formula I and
X is Cl, Br, I, triflate or nonaflate, with a compound of formula III 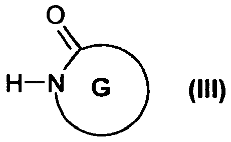 wherein ring G are as defined in formula I,
in the presence of a metal catalyst, selected from palladium and copper, a base, a ligand, wherein the ligand is selected out of the group (+/-)-2,2'-bis(diphenylphosphino)-1,1'-binaphthalene, (*R*)-(-)-1-[(*S*)-2-(diphenylphosphino) ferrocenyl] ethyldicyclohexylphosphine, 1,2-Bis(diphenylphosphino)ethane , 1,3-Bis(diphenylphosphino)propane, (*R*)-(-)-1-[(*S*)-2-(Dicyclohexylphosphino)-ferrocenyl]ethyldi-tert-butylphosphine, (*R*)-(+)-1,1'-Bis(diphenylphosphino)-2,2'-bis(*N*,*N*-diiisopropylamido)ferrocene, (*S*,*S*)-1-[1-(Di-tert-butylphosphino)ethyl]-2-(diphenylphosphino)ferrocene, (1*R*,2*R*)-(+)-1,2-Diaminocyclohexane-*N*,*N*'-bis(2-diphenylphosphino-1-naphtoyl, (-)-1,2-Bis((2*S*,5*S*)-2,5-diiso-propylphospholano)-benzene, Bis[(2-diphenylphosphino)phenyl]ether, (*S*)-(-)-2,2'-Bis(di-paratolylphosphino)-1,1'-binaphyl, 4,5-Bis(bis(3,5-bis(trifluoromethyl)phenyl)-phosphino)-9,9-dimethylxanthen, 2,2'-bis[(2',4',6'-triisopropyl)dicyclohexylphosphino]biphenyl and 2,2'-bis(di-*tert*-butylphosphino)biphenyl, tri-*tert-*butylphosphine, if the metal catalyst is palladium or
the ligand is selected out of the group ethylenediamine, *N*-methylethylenediamine, *N,N*-dimethyl-ethane-1,2-diamine *N*-buthylethylenediamine, *N*,*N-*dimethylethylenediamine, *N*,*N*,*N'*-trimethylthylenediamine, *N,N,N,N'-*tetramethylthylenediamine, *trans*-1,2-cyclohexanodiamine, cis-1,2-cyclohexanodiamine, *cis*/*trans*-1,2-cyclohexanodiamine, *N,N*'-diethyl-1,2-cyclohexanodiamine, *N*,*N*'-dipropyl-1,2-cyclohexanodiamine, 1,3-propylenediamine, 1,2-benzenediamine, phenanthridine, acridine, acridine orange, 9-aminoacridine, 9-hydroxy-4-methoxyacridine, proflavine, 4-(2-pyriylazo) resorcinol, 1,2-dihydro-1-(2-(2-pyridyl)-ethyl)-3,6-pyridazinedione, [1,10]phenanthroline, 5-nitro-[1,10]phenanthroline, bathophenanthroline, spiramycin, bicinchonic acid sodium salt (bca), 1-(4-pyridyl) pyridinium chloride, 2-pyridylacetic acid hydrochloride, 8-mercaptoquinoline hydrochloride, dimethylamino acetic acid, picolinic acid, 3-hydroxypicolinic acid, 3-hydroxy picolinamide, glycol, pyridine, 2-aminopyridine, 2-hydroxipyridine, 3-cyanopyridine, 4-cyanopyridine, 2-ethylpyridine, 2-amino-6-methylpyridine, 2-(aminomethylpyridine), 2-(hydroximethylpyridine), 2-hydroxi-6-methylpyridine, 2-dimethylaminopyridine, 4-dymethylaminopyridine, 2-(2-hydroxiethyl)pyridine, 4-tert-butylpyridine, 3-acetoxypyridine, 2-phenylpyridine, 4-phenylpyridine, 4-benzoylpyridine, 2-(2-thienyl)pyridine, 2-benzylpyridine, 2-anilinopyridine, 3-pyridinepropanol, 1-(2-pyridyl) piperazine, di-2-pyridyl ketone, ethyl 2-pyridyl acetate, 2-(2-diethylaminoethyl)-pyridine, 4-(2-diethylaminoethyl)pyridine, 2,6-di-tert-butyl pyridine, (*S*,*S*)-2,6-bis(4-isopropyl-2-oxazolin-2-yl) pyridine, 2,3-pyridine dicarboxylic acid, 2,6-pyridine dicarboxylic acid, 3,5-pyridine dicarboxylic acid, 1,3-di(4-pyridyl)propane, 2,3-di-3-pyridyl-2,3-butanediol, 2,2'-bipyridine, 2,2-dipyridyl, 4,4'-dimethyl-2,2'-dipyridyl, 3-hydroxypyridine, 2-mercaptopyridine, 2-(2-methylaminoethyl) pyridine, 3-hydroxi picolinamine, 3-hydroxypicolinic acid, 2,2':6',2"-terpyridine, 2-picoline, 6,6'-bi-2-picoline, 2,4-lutidine, 2,6-lutidine-α-2,3-diol, 2,6-lutidine 2,4,6-collidine, picolinamide, ethyl picolinate, ethyl isonicotinate, quinoline, 2-phenylquinoline, 8-hidroxyquinoline, 8-acetoxyquinoline, 2-methyl-8-nitroquinoline, 7,8-benzoquinoline, 2-quinolinol, 2-quinolinethiol, quinoline-4-carboxylic acid, 2-phenyl-4-quinoline carboxylic acid, 2,4-hydroxy quinoline monosodium salt, 8-ethoxyquinoline-5-sulfonic acid sodium salt, 8-hydroxy-5-nitroquinoline, 4-chloro-7-(trifluoromethyl) quinoline, 8-hydroxyquinoline-5-sulfonic acid monohydrate, 5-nitroquinaldic acid, isoquinoline, isoquinoline-3-carboxylic acid hydrate, 1,4,5-triazanaphtalene, quinaldine, 4-chloroquinaldine, nicotine, isonicotinamine, neocuproine, lysine, cystine, α-alanine, arginine, cysteine and β-alanine, if the metal catalyst is copper
and an aprotic solvent to give a compound of formula IV 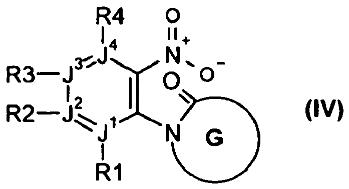 and converting the compound of formula IV into a compound of formula I in the presence of a reducing reagent and a second solvent and
optionally the compound of formula I is converted to its physiologically tolerated salt.

The present invention also relates to a process for the preparation of a compound of formula I, wherein
J1, J2, J3 and J4 form together with the carbon atoms they are attached to a ring selected from benzene, pyrazine, pyridazine, pyridine, pyrimidine, triazine or tetrazine,
G is selected from azetidine, azepane, azocane, aza-bicyclo[2.2.1]heptane, azabicyclo[2.2.2]octane, azacyclooctanone, azacyclononanone, aza-tricyclo [4.3.1.1*3,8*]undecane, 4,4-dimethyl-3,5-dioxa-azatricyclo[5.2.1.0*2,6*]-decane, 3,5-dioxa-azatricyclo-[5.2.1.0*2,6*]decane, 4,4-dimethyl-3,5-dioxa-azatricyclo [5.2.1.0*2,6*]decan-9-one, azocane-2-one, azonane, 1,4-diazepane, [1,4]diazocane, [1,2]diazocan-3-one, [1,3]diazocan-2-one, imidazoline, imidazolidine, isothiazolidine, isoxazolidine, ketopiperazine, morpholine, [1,4]oxazocane, [1,3]oxazocan-2-one, piperazine, piperidine, pyrazoline, pyrazolidine, 1,2-dihydro-pyridine, pyrrolidine, pyrrolidinone, 2,3-dihydro-1H-pyrrole, pyrroline, 5,6,7,8-tetrahydro-1H-azocin-2-one, tetrahydropyridine, thiadiazine, thiazolidine, thiazoline or thiomorpholine, wherein G is unsubstituted or mono-, di-, tri- or four times substituted independently of one another by oxo or by R5,
R1, R2, R3, R4 and R5 are independent of one another identical or different and are
a) hydrogen atom,
b) F,
c) Cl,
d) -(C₁-C₄)-alkyl, wherein alkyl is unsubstituted or substituted one to three times by R13,
e) -(C₁-C₃)-fluoroalkyl,
f) phenyl, wherein phenyl is unsubstituted or substituted one to three times by R13,
g) -(C₄-C₁₄)-heteroaryl, wherein heteroaryl is selected from acridinyl, azaindole (1H-pyrrolopyridinyl), azabenzimidazolyl, azaspirodecanyl, azepinyl, azetidinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo[1,3,4]oxathiazinyl, 6H-1,5,2-dithiazinyl, dihydrofuro[2,3-b]-tetrahydrofuranyl, furanyl, furazanyl, imidazolidinyl, imidazolinyl, imidazolyl, indanyl, 1 H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyrazolo[3,4-blpyridine, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,3-triazolyl, 1,2,4-triazolyl, 1,2,5-triazolyl, 1,3,4-triazolyl and xanthenyl,, and is unsubstituted or mono-, di-, tri- or four times substituted independently of one another by R13,
h) -(C₃-C₈)-cycloalkyl, wherein said cycloalkyl is unsubstituted or mono-, di-, tri- or four times substituted independently of one another by R13, or
i) a 3- to 7-membered cyclic residue is selected from azepine, azetidine, aziridine, azirine, 1,4 diazepane, 1,2-diazepine, 1,3-diazepine, 1,4-diazepine, diaziridine, diazirine, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketomorpholine, ketopiperazine, morpholine, 1,2-oxa-thiepane, 1,2-oxathiolane, 1,4-oxazepane, 1,2-oxazine, 1,3-oxazine, 1,4-oxazine, oxazole, oxaziridine, oxetan, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, and is unsubstituted or mono-, di-, tri- or four times substituted independently of one another by R13,
j) -O-CF₃,
k) -O-(C₁-C₄)-alkyl, wherein alkyl is unsubstituted or substituted one to three times by R13,
l) -N(R10)-(C₁-C₄)-alkyl, wherein alkyl is unsubstituted or substituted one to three times by R13,
m) -CN,
n) -OH,
o) phenyloxy-, wherein phenyloxy is unsubstituted or substituted one to three times by R13,
p) -C(O)-O-R11,
q) -C(O)-N(R11)-R12,
r) -N(R11)-R12,
s) -N(R10)-SO₂-R10,
t) -S-R10,
v) -SOₙ-R10, wherein n is 1 or 2,
w) -SO₂-N(R11)-R12,
x) -C(O)-R10 or
y) at least one of R1, R2, R3 or R4 are absent in case one or more of J1, J2, J3 or J4 are nitrogen atom,
R10 is hydrogen atom, -(C₁-C₃)-fluoroalkyl or -(C₁-C₆)-alkyl,
R11 and R12 are independently of one another identical or different and are
a) hydrogen atom,
b) -(C₁-C₄)-alkyl, wherein alkyl is unsubstituted or mono-, di- or trisubstituted independently of one another by R13,
c) phenyl, wherein phenyl is unsubstituted or mono-, di- or trisubstituted independently of one another by R13,
d) -(C₄-C₁₄)-heteroaryl, wherein heteroaryl is as defined above and is unsubstituted or mono-, di- or trisubstituted independently of one another by R13 or
R13 is F, Cl, -CN, =O, -OH, -(C₁-C₈)-alkyl, -(C₁-C₈)-alkoxy, -CF₃, phenyloxy-, -C(O)-R10, -C(O)-O-R17, -C(O)-N(R17)-R18, -N(R17)-R18, -N(R10)-SO₂-R10, -S-R10, -SOₙ-R10, wherein n is 1 or 2, -SO₂-N(R17)-R18, phenyl, wherein phenyl is unsubstituted or mono-, di- or trisubstituted independently of one another by R14, -(C₄-C₁₄)-heteroaryl, wherein heteroaryl is as defined above and is unsubstituted or mono-, di- or trisubstituted independently of one another by R14, -(C₃-C₆)-cycloalkyl, wherein said cycloalkyl is unsubstituted or mono-, di- or trisubstituted independently of one another by R14, or a 3- to 7-membered cyclic residue, which is as defined above and is unsubstituted or mono-, di- or trisubstituted independently of one another by R14, R14 is F, Cl, -OH, =O, -CN, -CF₃, -(C₁-C₈)-alkyl, -(C₁-C₄)-alkoxy, -C(O)-OH, -NH₂, -C(O)-O-(C₁-C₄)-alkyl, -(C₁-C₈)-alkylsulfonyl, -C(O)-NH₂, -C(O)-NH-(C₁-C₈)-alkyl, -C(O)-N-[(C₁-C₈)-alkyl]₂, -S-R10, -N(R10)-C(O)-NH-(C₁-C₅)-alkyl or-N(R10)-C(O)-N-[(C₁-C₈)-alkyl]₂,
R17 and R18 are independently of one another identical or different and are
a) hydrogen atom,
b) -(C₁-C₄)-alkyl,
c) phenyl or
d) -(C₄-C₁₄)-heteroaryl, wherein heteroaryl is as defined above and
X is Cl, Br or I.

The invention also relates to a process for the preparation of a compound of formula I, which are 2,3-Dihydro-1*H*-benzo[d]pyrrolo[1,2-a]imidazole;
7-Methyl-2,3-dihydro-1*H*-benzo[d]pyrrolo[1,2-a]imidazole;
6-Methyl-2,3-dihydro-1*H*-benzo[d]pyrrolo[1,2-a]imidazole;
7-Methoxy-2,3-dihydro-1*H*-benzo[d]pyrrolo[1,2-a]imidazole;
5-Methyl-2,3-dihydro-1*H* benzo[d]pyrrolo[1,2-a]imidazole-6-carboxylic acid methyl ester;
2-Methoxy-7,8-dihydro-6*H*-pyrrolo[2',1':2,3]imidazo[4,5-b]pyridine; 2,6-Dimethyl-7,8-dihydro-6*H*-pyrrolo[2',1':2,3]imidazo[4,5-b]pyridine; 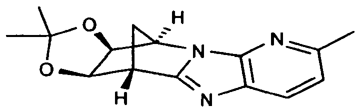 1,2,3,4-Tetrahydro-benzo[4,5]imidazo[1,2-a]pyridine;
3,9-Dimethyl-6,7,8,9-tetrahydro-dipyrido[1,2-a;3',2'-d]imidazole;
7-Chloro-4,4-diphenyl-1,2,3,4-tetrahydro-benzo[4,5]imidazo[1,2-a]pyridine;
Dimethyl-(*S*)-7,8,9,10-tetrahydro-6*H*-benzo[4,5]imidazo[1,2-a]azepin-6-yl-amine;
3-Methyl-5,6,7,8,9,10-hexahydro-4,4b,11-triaza-cycloocta[a]indene;
2-Methyl-6,7,8,9,10,11-hexahydro-5*H*-4,4b,12-triaza-cyclonona[a]indene;
3-Methyl-8,8-diphenyl-6,7,8,9-tetrahydro-dipyrido[1,2-a;3',2'-d]imidazole; 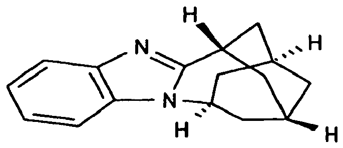 2-Methyl-8,8-diphenyl-6,7,8,9-tetrahydro-dipyrido[1,2-a:3',2'-d]imidazole;
5,6,7,8,9,10-Hexahydro-1,4b,11-triaza-cyclooct[a]indene; or
3-Methoxy-6,7,8,9,10,11-hexahydro-5*H*-4b,12-diaza-cyclonon[a]indene.

The aprotic solvent useful for step 1 in the process of the present invention must be solvent, wherein the compounds of formulae II, III and IV, metal catalyst, base and ligand are soluble or at least partially soluble and compatible and is chemically inert under the reaction conditions and does not contain water or oxygen as impurities. Examples of said aprotic solvents are: benzene, toluene, xylene, mesitylene, acetonitrile, tetrahydrofurane, dimethylformamide, n-methylpyrrolodinone, dimethylacetamide, dimethylsulfoxide, diglyme ((2-methoxyethyl)ether) or pyridine. Preferred is benzene, mesitylene or toluene. Most preferred is toluene.

The base useful in this process of the present invention is a basic organic or inorganic compound and acts as proton acceptor without inhibiting the catalytic activity of the employed metal catalyst e.g. palladium or copper species or preventing the coupled intermediate species of the compound of formula IV to undergo the reductive aminocyclisation. Suitable classes of such bases are for example carbonates, phosphates, fluorides, alkoxides and hydroxides with a suitable metal as counter ion. Carbonates and phosphates are the preferred bases in the process of the present invention. Potassium carbonate or potassium phosphate and in particular caesium carbonate are the preferred bases.

The bases are generally employed in moderate excess based on the 2-halo-nitroarene of the compound of formula II. A useful range is a 1.1 to 2 fold excess based on the 2-halo-nitroarene of the compound of formula II. The base may be favourably employed in a 1.4 fold excess based on the 2-halo-nitroarene of the compound of formula I.

The palladium catalyst useful in this process can be selected from the following classes: Pd-alkanoates, Pd-alkanoate complexes, Pd-acetonates, Pd-halides, Pd-halide complexes, Pd-phosphine complexes. Representative examples include, but are not limited to: palladium (II) acetate, palladium (II) trifluoroacetate, tris(dibenzylideneacetone)dipalladium(0), tris(dibenzylideneacetone)dipalladium(0) chloroform adduct, palladium (II) chloride, 2,2'-bis(diphenylphosphino)-1,1'-binaphthylpalladium(II) chloride, acetato(2'-di-tert-butylphosphino-1,1'-biphenyl-2-yl)palladium(II), (1,2-Bis(diphenylphosphino)ethane)dichloropalladium(II), Bis[1,2-bis(diphenylphosphino)ethane]palladium (0), [(2S,3S)-Bis(diphenylphosphino)butane] [eta3-allyl]palladium(II) perchlorate, 1,3-bis(2,4,6-trimethylphenyl)imidazol-2-ylidene(1,4-naphthoquinone)palladium (0) dimer. The preferred catalysts are palladium (II) acetate, 2,2'-bis(diphenylphosphino)-1,1'-binaphthylpalladium(II) and in particular palladium (II) trifluoroacetate.

The palladium catalyst is generally employed in an amount in the range of 1 to 10 mole percent based on the 2-halo-nitroarene of the compound of formula II. A useful range is 1 to 9 mole percent of palladium catalyst based on the 2-halo-nitroarene of the compound of formula I.

The phosphine ligand is generally employed in an amount in the range of 1 to 10 mole percent based on the 2-halo-nitroarene of the compound of the compound of formula II. A useful range is 1 to 9 mole percent of phosphine ligand based on the 2-halo-nitroarene of the compound of formula II. Most favourably the phosphine ligand is employed in an equimolar ratio with respect to the palladium source.

The copper catalyst useful in this process can be selected from the following classes: copper (I) halogen salts and copper oxides. Representative examples include, but are not limited to: copper (I) chloride, copper (I) bromide, copper (I) iodide and copper (I) oxide. The preferred catalyst is copper (I) iodide.

The copper catalyst is generally employed in an amount in the range of 0.1 to 30 mole percent based on the 2-halo-nitroarene of the compound of formula II. A useful range is 1 to 9 mole percent of copper catalyst based on the 2-halo-nitroarene of the compound of formula I.

The amine ligand is generally employed in an amount in the range of 0.1 to 60 mole percent based on the 2-halo-nitroarene of the compound of the compound of formula II. A useful range is 5 to 15 mole percent of amine ligand based on the 2-halo-nitroarene of the compound of formula II. Most favourably the amine ligand is employed in a ratio of 2 with respect to the copper source.

The reaction step 1 is carried out in the temperature range 60 °C to 150°C. A useful temperature is from 90°C to 110°C, preferably from 70°C to 90 °C. Generally the reaction is carried out under the exclusion of air and moisture such as under an inert atmosphere like e.g. in an argon or nitrogen atmosphere at atmospheric pressure. The reaction time for step 1 is in the range of 3 to 48 hours (h).

It is possible to filtrate or to isolate the compound of formula IV before reacting it in the second step. It is also possible to perform reaction step 2 without any separation step in the same reaction vessel.

The solvent useful for step 2 or the second solvent in the process of the present invention is an aprotic or protic solvent, wherein the compounds of formula IV or I are soluble or at least partially soluble and compatible with the reaction conditions and involved structures and reagents. Examples of said aprotic or protic solvents are: methanol, ethanol, propanol, acetic acid, methylene chloride, dimethylformamide, tetrahydrofurane, pyridine, p-xylene, ethylacetate, benzene, toluene, xylene, mesitylene or acetonitrile. Preferred are methanol, ethanol, acetic acid, methylene chloride, dimethylformamide, pyridine, *p*-xylene and isopropanol. Most preferred is acetic acid.

The reducing reagent useful for the reductive aminocyclisation in step 2 in the process of the present invention can be selected from the following examples, but are not limited to: H₂/Raney-Ni, H₂/Pd-C, H₂/PtO₂, H₂/Ru, NaBH₄/NiCl₂, NaBH₄/FeCl₂, H₃PO₂/Pd-C, Sn/HCl, SnCl₂/HCl, Fe/HOAc, Fe/HCl, FeSO₄/HCl, Fe/FeSO₄, Zn/HCl, Na₂S, and Na₂S₂O₄. Favourable is Fe/HOAc as a reagent for the reductive aminocyclisation.

The reaction step 2 is carried out in the temperature range 80 °C to 140 °C. A useful temperature is from 110°C to 120 °C.

The reaction time for step 2 is in the range of 15 min to 120 min.

The progress of each reaction step may be monitored by methods known to those skilled in the art, like for example thin layer silica gel chromatography, gas chromatography, nuclear magnetic resonance, infrared spectroscopy, and high pressure liquid chromatography combined with ultraviolet detection or mass spectroscopy. Preferably thin layer silica gel chromatography and high pressure liquid chromatography (HPLC) combined with mass spectroscopy are used.

The isolation and purification procedures useful for the compounds obtained by the process of the present invention are well-known to those skilled in the art, like for example filtration through a celite containing cartridge, aqueous work-up, extraction with organic solvents, distillation, crystallisation, chromatography on silica, and high pressure liquid chromatography on normal phase or reversed phase. Preferred methods include, but are not limited to those exemplified.

The term alkyl as used herein expressly includes saturated groups as well as unsaturated groups which latter groups contain one or more, for example one, two or three, double bonds and/or triple bonds. All these statements also apply if an alkyl group occurs as a substituent on another residue, for example in an alkyloxy residue, an alkyloxycarbonyl residue or an arylalkyl residue. Examples of "(C₁-C₈)-alkyl" or "-(C₁-C₈)-alkylene" are alkyl residues containing 1, 2, 3, 4, 5, 6, 7 or 8 carbon atoms are methyl, methylene, ethyl, ethylene, propyl, propylene, butyl, butylene, pentyl, pentylene, hexyl, heptyl or octyl, the n-isomers of all these residues, isopropyl, isobutyl, 1-methylbutyl, isopentyl, neopentyl, 2,2-dimethylbutyl, 2-methylpentyl, 3-methylpentyl, isohexyl, sec-butyl, tBu, tert-pentyl, sec-butyl, tert-butyl or tert-pentyl. Unsaturated alkyl residues are e.g. alkenyl residues such as vinyl, 1-propenyl, 2-propenyl (= allyl), 2-butenyl, 3-butenyl, 2-methyl-2-butenyl, 3-methyl-2-butenyl, 5-hexenyl or 1,3-pentadienyl, or alkynyl residues such as ethynyl, 1-propynyl, 2-propynyl (= propargyl) or 2-butynyl. Alkyl residues can also be unsaturated when they are substituted.

The term "-(C₃-C₈)-cycloalkyl" is understood as cyclic alkyl residues are cycloalkyl residues containing 3, 4, 5, 6, 7 or 8 ring carbon atoms like cyclopropyl, cyclobutyl, cyclopentyl, cyclohexyl, cyloheptyl or cyclooctyl, which can also be substituted and/or unsaturated. Unsaturated cyclic alkyl groups and unsaturated cycloalkyl groups like, for example, cyclopentenyl or cyclohexenyl can be bonded via any carbon atom.

The term "J1, J2, J3 and J4 are independently from each other selected from carbon or nitrogen atoms and form together with the carbon atoms they are attached to a stable aromatic or heteroaromatic ring" refers to a residue which can be derived from compounds such as benzene pyrazine, pyridazine, pyridine, pyrimidine, triazine or tetrazine.

The term "-(C₆-C₁₄)-aryl" is understood as meaning aromatic hydrocarbon radicals containing from 6 to 14 carbon atoms in the ring. Examples of -(C₆-C₁₄)-aryl radicals are phenyl, naphthyl, for example 1-naphthyl and 2-naphthyl, biphenylyl, for example 2-biphenylyl, 3-biphenylyl and 4-biphenylyl, anthryl or fluorenyl. Biphenylyl radicals, naphthyl radicals and, in particular, phenyl radicals are preferred aryl radicals.

The term monocyclic, bicyclic or tricyclic 4- to 15-membered saturated, or partially unsaturated heterocyclic ring containing in addition to the nitrogen atom of the lactam moiety 1, 2, 3 or 4 heteroatoms chosen from nitrogen, sulfur or oxygen" refers to any monocyclic or bicyclic 4- to 15-membered heterocyclic ring system containing up to 1, 2, 3 or 4 heteroatoms like for example selected from azetidine, azepane, azocane, aza-bicyclo[2.2.1]heptane, aza-bicyclo[2.2.2]octane, azacyclooctanone, azacyclononanone, aza-tricyclo [4.3.1.1*3,8*]undecane, 4,4-dimethyl-3,5-dioxaazatricyclo[5.2.1.0*2,6*]-decane, 3,5-dioxa-azatricyclo-[5.2.1.0*2,6*]decane, 4,4-dimethyl-3,5-dioxa-azatricyclo [5.2.1.0*2,6*]decan-9-one, azocane-2-one, azonane, 1,4-diazepane, [1,4]diazocane, [1,2]diazocan-3-one, [1,3]diazocan-2-one, imidazoline, imidazolidine, isothiazolidine, isoxazolidine, ketopiperazine, morpholine, [1,4]oxazocane, [1,3]oxazocan-2-one, piperazine, piperidine, pyrazoline, pyrazolidine, 1,2-dihydro-pyridine, pyrrolidine, pyrrolidinone, 2,3-dihydro-1H-pyrrole, pyrroline, 5,6,7,8-tetrahydro-1H-azocin-2-one, tetrahydropyridine, thiadiazine, thiazolidine, thiazoline or thiomorpholine.

The term "-(C₄-C₁₄)-heteroaryl" refers to mono-, di- or tri-ring systems, wherein one or more of the 4 to 14 ring carbon atoms are replaced by heteroatoms such as nitrogen, oxygen or sulfur. Examples are acridinyl, azaindole (1 H-pyrrolopyridinyl), azabenzimidazolyl, azaspirodecanyl, azepinyl, azetidinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo[1,3,4]oxathiazinyl, 6H-1,5,2-dithiazinyl, dihydrofuro[2,3-b]-tetrahydrofuranyl, furanyl, furazanyl, imidazolidinyl, imidazolinyl, imidazolyl, indanyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,3-triazolyl, 1,2,4-triazolyl, 1,2,5-triazolyl, 1,3,4-triazolyl and xanthenyl. The term "a 3- to 7-membered cyclic residue, containing 1, 2, 3 or 4 heteroatoms" refer to structures of heterocycles, which can be derived from compounds such as azepine, azetidine, aziridine, azirine, 1,4 diazepane, 1,2-diazepine, 1,3-diazepine, 1,4-diazepine, diaziridine, diazirine, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketomorpholine, ketopiperazine, morpholine, 1,2-oxa-thiepane, 1,2-oxathiolane, 1,4-oxazepane, 1,2-oxazine, 1,3-oxazine, 1,4-oxazine, oxazole, oxaziridine, oxetan, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole.

The 3- to 7- membered monocyclic group may be bonded via any ring carbon atom, and in the case of nitrogen heterocycles via any suitable ring nitrogen atom. Thus, for example, a pyrrolyl residue can be 1-pyrrolyl, 2-pyrrolyl or 3-pyrrolyl, a pyrrolidinyl residue can be pyrrolidin-1-yl (= pyrrolidino), pyrrolidin-2-yl or pyrrolidin-3-yl, a pyridinyl residue can be pyridin-2-yl, pyridin-3-yl or pyridin-4-yl, a piperidinyl residue can be piperidin-1-yl (= piperidino), piperidin-2-yl, piperidin-3-yl or piperidin-4-yl. Furyl can be 2-furyl or 3-furyl, thienyl can be 2-thienyl or 3-thienyl, imidazolyl can be imidazol-1-yl, imidazol-2-yl, imidazol-4-yl or imidazol-5-yl, 1,3-oxazolyl can be 1,3-oxazol-2-yl, 1,3-oxazol-4-yl or 1,3-oxazol-5-yl, 1,3-thiazolyl can be 1,3-thiazol-2-yl, 1,3-thiazol-4-yl or 1,3-thiazol-5-yl, pyrimidinyl can be pyrimidin-2-yl, pyrimidin-4-yl (= 6-pyrimidinyl) or 5-pyrimidinyl, piperazinyl can be piperazin-1-yl (= piperazin-4-yl = piperazino) or piperazin-2-yl.

The term "R1 and R2, R2 and R3 or R3 and R4 form together with the atoms which they are attached to a 5- or 8-membered ring, containing up to 0, 1, 2, 3 or 4 heteroatoms chosen from nitrogen, sulfur or oxygen" refers to residues which can be derived from compounds such as azepine, azirine, azocane, azocane-2-one, cyloheptyl, cyclohexyl, cyclooctane, cyclooctene, 1,4-diazepane, 1,2-diazepine, 1,3-diazepine, 1,4-diazepine, [1,2]diazocan-3-one, [1,3]diazocan-2-one, [1,4]diazocane, dioxazine, dioxazole, [1,4]dioxocane, 1,3-dioxolane, dioxole, 1,3-dioxolene, furan, imidazole, imidazolidine, imidazoline, isothiazole, isothiazolidine, isothiazoline, isothiazole, isoxazole, isoxazolidine, isoxazoline, 2-isoxazoline, ketomorpholine, ketopiperazine, morpholine, 1,2-oxa-thiepane, 1,2-oxathiolane, 1,4-oxazepane, 1,2-oxazine, 1,3-oxazine, 1,4-oxazine, oxaziridine,[1,4]oxazocane, [1,3]oxazocan-2-one, oxocane, oxocan-2-one, oxazole, piperidine, piperazine, phenyl, pyridazine, pyridine, pyrimidine, pyran, pyrazine, pyrazole, pyrazolepyrrole, pyrazolidine, pyrazoline, pyridazine, pyridine, pyrimidine, pyrrole, pyrrolidine, pyrrolidinone, pyrroline, 5,6,7,8-tetrahydro-1H-azocin-2-one, tetrahydrofuran, tetrahydropyran, tetrahydropyridine, tetrazine, tetrazole, thiadiazine, thiadiazole, 1,2-thiazine, 1,3-thiazine, 1,4-thiazine, thiazole, 1,3-thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole.

The fact that many of the before-listed names of heterocycles are the chemical names of unsaturated or aromatic ring systems does not imply that the, 4- to 14-membered mono- or polycyclic group could only be derived from the respective unsaturated ring system. The names here only serve to describe the ring system with respect to ring size and the number of the heteroatoms and their relative positions. As explained above, the 4- to 14-membered mono- or polycyclic group can be saturated or partially unsaturated or aromatic, and can thus be derived not only from the before-listed heterocycles themselves but also from all their partially or completely hydrogenated analogues and also from their more highly unsaturated analogues if applicable. As examples of completely or partially hydrogenated analogues of the before-listed heterocycles from which this group may be derived the following may be mentioned: pyrroline, pyrrolidine, tetrahydrofuran, tetrahydrothiophene, dihydropyridine, tetrahydropyridine, piperidine, 1,3-dioxolane, 2-imidazoline, imidazolidine, 4,5-dihydro-1,3-oxazol, 1,3-oxazolidine, 4,5-dihydro-1,3-thiazole, 1,3-thiazolidine, perhydro-1,4-dioxane, piperazine, perhydro-1,4-oxazine (= morpholine), perhydro-1,4-thiazine (= thiomorpholine), perhydroazepine, indoline, isoindoline, 1,2,3,4-tetrahydroquinoline or 1,2,3,4-tetrahydroisoquinoline.

The term "-(C₁-C₃)-fluoroalkyl" is a partial or totally fluorinated alkyl-residue, which can be derived from residues such as -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CFg, -CF₂-CF₂-CHF₂ or -CF₂-CF₂-CH₂F.

Halogen is fluorine, chlorine, bromine or iodine, preferably fluorine, chlorine or bromine, particularly preferably chlorine or bromine.

The term "triflate" refers to trifluoro-methanesulfonic acid ester or trifluoromethanesulfonate.

The term "nonaflate" refers to 1,1,2,2,3,3,4,4,4-nonafluoro-1-butanesulfonic acid ester or 1,1,2,2,3,3,4,4,4-nonafluoro-1-butanesulfonate.

The term "at least one of R1, R2, R3 or R4 are absent in case one or more of J1, J2, J3 or J4 are nitrogen atom," refers to a residue wherein the nitrogen atom is not substituted by any residue, e.g. in case J1 is nitrogen atom and J2, J3 and J4 are each a carbon atom and R4 is absent and R1, R2 and R3 are each a hydrogen atom the residue pyridine is formed. If R1, R2 and R3 are not each a hydrogen atom but one of the residues specified under b) to x) then a substituted pyridine residue is formed. In case J1 and J2 are each a nitrogen atom and J3 and J4 are each a carbon atom and R4 and R3 are absent and R1 and R2 are each a hydrogen atom the residue pyridazine is formed. If R1 and R2 are not each a hydrogen atom but one of the residues specified under b) to x) then a substituted pyridazine residue is formed.

Optically active carbon atoms present in the compounds of the formula (I) can independently of each other have R configuration or S configuration. The compounds of the formula (I) can be present in the form of pure enantiomers or pure diastereomers or in the form of mixtures of enantiomers and/or diastereomers, for example in the form of racemates. The present invention relates to pure enantiomers and mixtures of enantiomers as well as to pure diastereomers and mixtures of diastereomers. The invention comprises mixtures of two or of more than two stereoisomers of the formula (I), and it comprises all ratios of the stereoisomers in the mixtures. In case the compounds of the formula (I) can be present as E isomers or Z isomers (or cis isomers or trans isomers) the invention relates both to pure E isomers and pure Z isomers and to E/Z mixtures in all ratios. The invention also comprises all tautomeric forms of the compounds of the formula (I).

Diastereomers, including E/Z isomers, can be separated into the individual isomers, for example, by chromatography. Racemates can be separated into the two enantiomers by customary methods, for example by chromatography on chiral phases or by resolution, for example by crystallization of diastereomeric salts obtained with optically active acids or bases. Stereochemically uniform compounds of the formula (I) can also be obtained by employing stereochemically uniform starting materials or by using stereoselective reactions.

The starting materials or building blocks for use in the general synthetic procedures that can be applied in the preparation of the compounds of formula (I) are readily available to one of ordinary skill in the art. In many cases they are commercially available or have been described in the literature. Otherwise they can be prepared from readily available precursor compounds analogously to procedures described in the literature, or by procedures or analogously to procedures described in this application.

Further, in order to obtain the desired substituents in the benzene nucleus and in the heterocyclic nucleus of the benzimidazole or azabenzimidazole ring system in the formula (I), the functional groups introduced into the ring system during the benzimidazole or azabenzimidazole synthesis can be chemically modified. For example, benzimidazoles carrying a hydrogen atom in the 7-position can also be obtained by oxidation of 7-methyl benzimidazole to the benzimidazole-7-carboxylic acid and subsequent decarboxylation or from benzimidazoles carrying an ester group in the respective position. Carboxylic acid groups and acetic acid groups in the 7-position can be converted into their homologues by usual reactions for chain elongation of carboxylic acids.

Especially the groups present in the benzimidazole or azabenzimidazole ring system can be modified by a variety of reactions and thus the desired residues R1, R2, R3, R4 and R5 be obtained. For example, nitro groups can be reduced to amino group with under the described reaction conditions or by various reducing agents, such as sulfides, dithionites, complex hydrides or by catalytic hydrogenation. A reduction of a nitro group may also be carried out at a later stage of the synthesis of a compound of the formula (I), and a reduction of a nitro group to an amino group may also occur simultaneously with the reaction performed on another functional group, for example when reacting a group like a cyano group with hydrogen sulfide or when hydrogenating a group. Ester groups present in the benzene nucleus can be hydrolyzed to the corresponding carboxylic acids, which after activation can then be reacted with amines or alcohols under standard conditions. Ether groups present at the benzene nucleus, for example benzyloxy groups or other easily cleavable ether groups, can be cleaved to give hydroxyl groups which then can be reacted with a variety of agents, for example etherification agents or activating agents allowing replacement of the hydroxyl group by other groups. Sulfur-containing groups can be reacted analogously.

Due to the fact that in the present case the functional groups are attached to an benzimidazole or azabenzimidazole ring it may in certain cases become necessary to specifically adapt reaction conditions or to choose specific reagents from a variety of reagents that can in principle be employed into a conversion reaction, or otherwise to take specific measures for achieving a desired conversion, for example to use protection group techniques. However, finding out suitable reaction variants and reaction conditions in such cases does not cause any problems for one skilled in the art.

In the course of the preparation of the compounds of the formula I it can generally be advantageous or necessary to introduce functional groups which reduce or prevent undesired reactions or side reactions in the respective synthesis step, in the form of precursor groups which are later converted into the desired functional groups, or to temporarily block functional groups by a protective group strategy suited to the synthesis problem. Such strategies are well known to those skilled in the art (see, for example, Greene and Wuts, Protective Groups in Organic Synthesis, Wiley, 1991, or P. Kocienski, Protecting Groups, Thieme 1994). As example of a precursor group cyano groups may be mentioned which can in a later step be transformed into carboxylic acid derivatives or by reduction into aminomethyl groups. Protective groups can also have the meaning of a solid phase, and cleavage from the solid phase stands for the removal of the protective group. The use of such techniques is known to those skilled in the art (Burgess K (Ed.) Solid Phase Organic Synthesis ,New York: Wiley, 2000). For example, a phenolic hydroxy group can be attached to a trityl-polystyrene resin, which serves as a protecting group, and the molecule is cleaved from this resin by treatment with trifluoroacetic acid (TFA) at a later stage of the synthesis.

In the course of the synthesis the employment of microwave assistance for speeding-up, facilitating or enabling reactions may be beneficial or even required in many cases. Some reactions are for example described by J. L. Kristenansky, I. Cotteril, Curr. Opin. Drug. Disc. & Development., 4(2000), 454; Lidstrom, J. Tierney, B. Wathey, J. Westman, Tetrahedron, 57(2001), 9225; M. Larhed, A. Hallberg, Drug Discovery Today, 8 (2001) 406; S. Caddick, Tetrahedron, 51 (1995) 10403.

Physiologically tolerable salts of the compounds of formula I are nontoxic salts that are physiologically acceptable, in particular, pharmaceutically utilizable salts. Such salts of compounds of formula I containing acidic groups, for example, a carboxyl group (COOH), include, for example, alkali metal salts or alkaline earth metal salts, such as sodium salts, potassium salts, magnesium salts and calcium salts, as well as salts with physiologically tolerable quaternary ammonium ions, such as tetramethylammonium or tetraethylammonium, and acid addition salts with ammonia and physiologically tolerable organic amines, such as methylamine, dimethylamine, trimethylamine, ethylamine, triethylamine, ethanolamine or tris-(2-hydroxyethyl)amine. Basic groups contained in the compounds of formula I, for example, amino groups or guanidino groups, form acid addition salts, for example, with inorganic acids such as hydrochloric acid, hydrobromic acid, sulfuric acid, nitric acid or phosphoric acid, or with organic carboxylic acids and sulfonic acids such as formic acid, acetic acid, oxalic acid, citric acid, lactic acid, malic acid, succinic acid, malonic acid, benzoic acid, maleic acid, fumaric acid, tartaric acid, methanesulfonic acid or p-toluenesulfonic acid. Compounds of the formula I which simultaneously contain a basic group and an acidic group, for example, a guanidino group and a carboxyl group, can also be present as zwitterions (betaines) which are likewise included in the scope of the present invention.

Salts of compounds of formula I can be obtained by customary methods known to those skilled in the art, for example, by combining a compound of the formula I with an inorganic or organic acid or base in a solvent or dispersant, or from other salts by cation exchange or anion exchange. The present invention also includes all salts of the compounds of formula I which, because of low physiologically tolerability, are not directly suitable for use in pharmaceuticals but are suitable, for example, as intermediates for carrying out further chemical modifications of the compounds of formula I or as starting materials for the preparation of physiologically tolerable salts.

A further aspect of the invention is the use of a compound of the formula I as prepared by the process according to the invention for the production of pharmaceuticals, diagnostic agents, liquid crystals, polymers, herbicides, fungicidals, nematicidals, parasiticides, insecticides, acaricides and arthropodicides.

Preferred methods include, but are not limited to those described in the examples. Furthermore, the compounds of the formula I can be used as synthesis intermediates for the preparation of other compounds, in particular of other pharmaceutical active ingredients, which are obtainable from the compounds of the formula I, for example by introduction of substituents or modification of functional groups.

The general synthetic sequences for preparing the compounds useful in the present invention are outlined in the examples given below. Both an explanation of, and the actual procedure for, the various aspects of the present invention are described where appropriate. The following examples are intended to be merely illustrative of the present invention, and not limiting thereof in either scope or spirit. Those with skill in the art will readily understand that known variations of the conditions and processes described in the examples can be used to synthesize the compounds of the present invention.

### Examples

When in the final step of the synthesis of a compound an acid such as trifluoroacetic acid or acetic acid was used, for example when trifluoroacetic acid was employed to remove a tBu group or when a compound was purified by chromatography using an eluent which contained such an acid, in some cases, depending on the work-up procedure, for example the details of a freeze-drying process, the compound was obtained partially or completely in the form of a salt of the acid used, for example in the form of the acetic acid salt or trifluoroacetic acid salt or hydrochloric acid salt.

**Abbreviations used:**

| | |
|---|---|
| 2,2'-bis(diphenylphosphino)-1,1'-binaphthalene | BINAP |
| Calculated | cal |
| dibenzylidenacetone | dba |
| Dimethylsulfoxide | DMSO |
| 1,1'-Bis(diphenylphosphino)ferrocene | DPPF |
| Fast atom bombardment | FAB |
| Acetic acid | HOAc |
| High pressure liquid chromatography | HPLC |
| Liquid chromatography with mass spectrometry | LC-MS |
| Melting point | mp |
| Room temperature 20°C to 25 °C | RT |
| tert-Butyl | tBu |
| Trifluoroacetic acid | TFA |
| 4,5-Bis(diphenylphosphino)-9,9-dimethylxanthene | Xantphos |

### Example 1: 2,3-Dihydro-1H-benzo[d]pyrrolo[1,2-a]imidazole

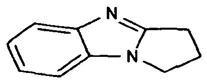

(Method A): 1-Iodo-2-nitrobenzene (125 mg, 0.5 mmol), pyrrolidin-2-one (51 mg, 0.6 mmol), palladium trifluoroacetate (13 mg, 0.04 mmol), BINAP (24 mg, 0.08 mmol), and cesium carbonate (212 mg, 0.7 mmol) were placed in a reaction tube, which was the purged with dry argon. Dry toluene (3 mL), was added, and the mixture was heated at 80°C for 18 h. After cooling to RT, 10 mL of glacial acetic acid and powder iron (279 mg, 5 mmol) were added and the crude was refluxed for 30 min. The acid was removed under reduced pressure and the residue was suspended in saturated sodium bicarbonate solution and extracted with ethyl acetate. The obtained crude was purified by preparative HPLC, affording the title compound as colorless solid (58 mg, 73%). mp 86-88-C. ¹H NMR δ 2.75 (t, *J* = 6.9 Hz, 2 H), 3.24-3.33 (m, 2 H), 4.33 (t, *J* = 7.2 Hz, 2 H), 7.46-7.50 (m, 2 H), 7.74-7.86 (m, 2 H); ¹³C NMR δ 23.7, 25.3, 45.3, 112.7, 115.1, 124.7, 125.0, 128.9, 136.6, 157.0. HRMS (FAB): calc. for C₁₀H₁₁N₂ [M+H⁺]: 159.0922; found: 159.0919.

### Example 2: 7-methyl-2,3-dihydro-1H-benzo[d]pyrrolo[1,2-a]imidazole

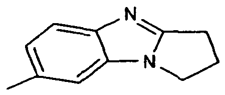

Method A applied to 2-Chloro-4-methyl-1-nitrobenzene (86 mg, 0.5 mmol) and pyrrolidin-2-one (51 mg, 0.6 mmol) afforded the title compound as a viscous oil (72 mg, 84%). ¹H NMR (DMSO) δ 2.73-2.82 (m, 2 H), 3.32 (t, *J* = 7.1 Hz), 4.35 (t, *J* = 7.2 Hz, 2 H), 7.33 (d, *J* = 8.3 Hz, 1 H), 7.57 (s, 1 H), 7.69 (d, *J* = 8.3 Hz, 1 H); ¹³C NMR δ 21.0, 23.8, 25.3, 45.6, 112.5, 114.3, 126.4, 127.1, 135.5, 136.1, 157.8.

### Example 3: 6-methyl-2,3-dihydro-1H-benzo[d]pyrrolo[1,2-a]imidazole

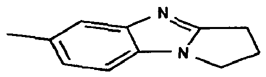

Method A applied to 1-Chloro-4-methyl-2-nitrobenzene (86 mg, 0.5 mmol) and pyrrolidin-2-one (51 mg, 0.6 mmol) afforded the title compound as a viscous oil (74 mg, 86%). ¹H NMR (DMSO) δ 2.55-2.63 (m, 2 H), 2.76 (s, 3H), 3.21 (t, *J* = 7.7 Hz, 2 H), 3.85 (s, 3 H), 4.26 (t, *J* = 7.1 Hz, 2 H), 7.56 (d, *J* = 8.6 Hz, 1 H), 7.85 (d, *J* = 8.6 Hz, 1 H); ¹³C NMR δ 21.0, 23.7, 25.2, 45.3, 112.4, 114.5, 126.5, 129.2, 134.8, 137.8, 157.2.

### Example 4: 7-methoxy-2,3-dihydro-1H-benzo[d]pyrrolo[1,2-a]imidazole

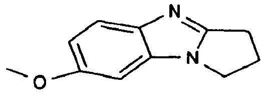

Method A applied to 1-Iodo-4-methoxy-2-nitrobenzene (140 mg, 0.5 mmol) and pyrrolidin-2-one (51 mg, 0.6 mmol) afforded the title compound as a viscous oil (84 mg, 89%). ¹H NMR (DMSO) δ 2.74 (t, *J* = 6.9 Hz, 2 H), 3.24-3.32 (m, 2 H), 3.86 (s, 3 H), 4.32 (t, *J* = 7.1 Hz, 2 H), 7.09 (d, *J* = 9.1 Hz, 1 H), 7.44 (d, *J* = 9.1 Hz, 1 H), 7.58 (s, 1 H); ¹³C NMR δ 23.6, 25.3, 45.4, 55.9, 96.0, 114.6, 115.6, 128.7, 129.6, 157.4, 158.2.

### Example 5: 5-Methyl-2,3-dihydro-1H-benzo[d]pyrrolo[1,2-a]imidazole-6-carboxylic acid methyl ester

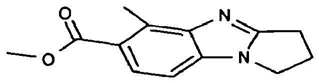

Method A applied to 4-Bromo-2-methyl-3-nitrobenzoic acid methyl ester (137 mg, 0.5 mmol) and pyrrolidin-2-one (51 mg, 0.6 mmol) afforded the title compound as a viscous oil (89 mg, 77%). ¹H NMR (DMSO) δ 2.55-2.63 (m, 2 H), 2.76 (s, 3 H), 3.21 (t, *J* = 7.7 Hz, 2 H), 3.85 (s, 3 H), 4.26 (t, *J* = 7.1 Hz, 2 H), 7.56 (d, *J* = 8.6 Hz, 1 H), 7.85 (d, *J* = 8.6 Hz, 1 H); ¹³C NMR δ 14.7, 23.4, 25.4, 44.5, 51.9, 99.1, 109.0, 124.0, 125.6, 129.2, 131.6, 161.6, 167.1.

### Example 6: 2-Methoxy-7,8-dihydro-6H-pyrrolo[2',1':2,3]imidazo[4,5-b]pyridine

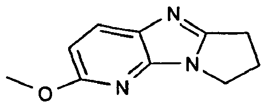

Method A applied to 2-Chloro-6-methoxy-3-nitropyridine (94 mg, 0.5 mmol) and pyrrolidin-2-one (51 mg, 0.6 mmol) afforded the title compound as a viscous oil (72 mg, 76%). ¹H NMR (DMSO) δ 2.68 (p, *J* = 6.9 Hz, 2 H), 3.23 (t, *J* = 6.9 Hz, 2 H), 3.91 (s, 3 H), 4.22 (t, *J* = 6.9 Hz), 6.82 (d, *J* = 8.6 Hz, 1 H), 8.02 (d, *J* = 8.6 Hz, 1 H).

### Example 7: 2,6-Dimethyl-7,8-dihydro-6H-pyrrolo[2',1':2,3]imidazo[4,5-b]pyridine

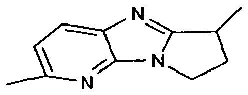

Method A applied to 2-Chloro-6-methyl-3-nitropyridine (94 mg, 0.5 mmol) and 3-methylpyrrolidin-2-one (59 mg, 0.6 mmol) afforded the title compound as a viscous oil (24 mg, 26%). ¹H NMR (DMSO) δ 1.42 (d, *J* = 6.9 Hz, 3 H), 2.22-2.32 (m, 1 H), 2.55 (s, 3 H), 2.86-2.97 (m, 1 H), 3.53-2.61 (m, 1 H), 4.12-4.38 (m, 2 H), 7.27 (d, *J* = 8.0 Hz, 1 H), 7.99 (d, *J* = 8.0 Hz, 1 H).

### Example 8:

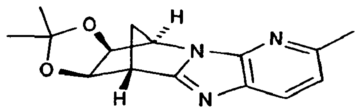

Method A applied to 2-Chloro-6-methyl-3-nitropyridine (94 mg, 0.5 mmol) and (1*S*,2*R*,6*S*,7*R*)-4,4-dimethyl-3,5-dioxa-8-azatricyclo[5.2.1.0*2,6*]decan-9-one (110 mg, 0.6 mmol) afforded the title compound as a viscous oil (126 mg, 88%). ¹H NMR (DMSO) δ 1.23 (s, 3 H), 1.51 (s, 3 H), 2.47-2.57 (m, 2 H), 2.53 (s, 3 H), 3.62 (s, 3 H), 4.23 (d, *J* = 4.8 Hz, 1 H). 4.35 (d, *J* = 4.8 Hz, 1 H), 7.12 (d, *J* = 7.9 Hz, 1 H), 7.88 (d, *J* = 7.9 Hz, 1 H).

### Example 9: 1,2,3,4-Tetrahydro-benzo[4,5]imidazo[1,2-a]pyridine

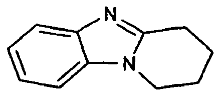

Method A applied to 1-Iodo-2-nitrobenzene (125 mg, 0.5 mmol) and piperidin-2-one (59 mg, 0.6 mmol) afforded the title compound as pale yellow solid (65 mg, 75%). mp 104-106°C. ¹H NMR (DMSO) δ 1.98-2.07 (m, 4 H), 3.16-3.23 (m, 2 H), 4.30 (t, *J* = 6.9 Hz, 2 H), 7.51-7.56 (m, 2 H), 7.75-7.92 (m, 2 H); ¹³C NMR δ 17.7, 20.5, 22.3, 43.0, 112.3, 114.2, 124.7, 125.6, 131.5, 151.8, 156.6. HRMS (FAB): cal for C₁₁H₁₃N₂ [M+H⁺]: 173.1079; found: 173.1071.

### Example 10: 3,9-Dimethyl-6,7,8,9-tetrahydro-dipyrido[1,2-a;3',2'-d]imidazole

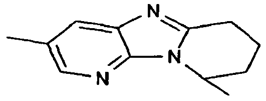

Method A applied to 2-Chloro-5-methyl-3-nitropyridine (86 mg, 0.5 mmol) and 3-methylpiperidin-2-one (68 mg, 0.6 mmol) afforded the title compound as viscous oil (60 mg, 60%). ¹H NMR (DMSO) δ 1.49 (s, 3 H), 1.51 (s, 3 H), 1.92-2.23 (m, 4 H), 3.04-3.20 (m, 2 H), 4.75-4.83 (m, 1 H), 7.98 (s, 1 H), 8.37 (s, 1 H).

### Example 11: 7-Chloro-4,4-diphenyl-1,2,3,4-tetrahydro-benzo[4,5]imidazo[1,2-a]pyridine

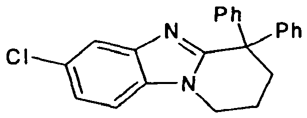

Method A applied to 2,5-Dichloronitrobenzene (96 mg, 0.5 mmol) and 3,3-diphenylpiperidin-2-one (151 mg, 0.6 mmol) afforded the title compound as a brown solid (63 mg, 35%). ¹H NMR (DMSO) δ 1.91-2.02 (m, 2 H), 2.76-2.81 (m, 2 H), 4.37 (t, *J* = 6.2 Hz, 2 H), 7.12-7.49 (m, 11 H), 7.64 (d, *J* = 8.8 Hz, 1 H), 7.69 (s, 1 H).

### Example 12: Dimethyl-(S)-7,8,9,10-tetrahydro-6H-benzo[4,5]imidazo[1,2-a]azepin-6-yl-amine

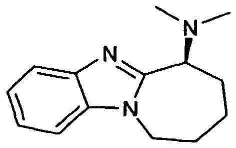

Method A applied to 1-Iodo-2-nitrobenzene (125 mg, 0.5 mmol) and (S)-3-dimethylaminoazepan-2-one (94 mg, 0.6 mmol) afforded the title compound as a pale yellow solid (84 mg, 73%). mp 164-166 °C. ¹H NMR (DMSO) δ 1.44-2.46 (m, 6 H), 3.03 (s, 6 H), 3.96 (dd, *J* = 11.9, 11.6 Hz, 1 H), 4.63 (dd, *J* = 14.5, 4.7 Hz, 1 H), 5.03 (d, *J* = 10.4 Hz, 1 H), 7.26 (t, *J* = 7.3 Hz, 1 H), 7.38 (t, *J* = 7.3 Hz, 1 H), 7.52-7.72 (m, 5 H), 7.79 (d, *J* = 7.8 Hz, 1 H); ¹³C NMR δ 25.2, 26.2, 27.0, 40.6, 43.5, 62.4, 110.3, 118.8, 122.0, 122.8, 135.3, 140.6, 151.1. HRMS (FAB): cal. for C₁₄H₂₀N₃ [M+H⁺]: 230.1657; found: 230.1648.

### Example 13: 3-Methyl-5,6,7,8,9,10-hexahydro-4,4b,11-triaza-cycloocta[a]indene

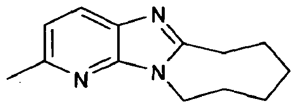

Method A applied to 2-Chloro-6-methyl-3-nitropyridine (94 mg, 0.5 mmol) and 2-azacyclooctanone (76 mg, 0.6 mmol) afforded the title compound as a viscous oil (74 mg, 69%). ¹H NMR (DMSO) δ 1.22-1.88 (m, 8 H), 2.62 (s, 3 H), 3.19 (t, *J* = 6.5 Hz, 2 H), 4.51 (t, *J* = 5.7 Hz, 2 H), 7.38 (d, *J* = 8.0 Hz, 1 H), 8.09 (d, *J* = 8.0 Hz, 1 H).

### Example 14: 2-Methyl-6,7,8,9,10,11-hexahydro-5H-4,4b,12-triaza-cyclonona[a]indene

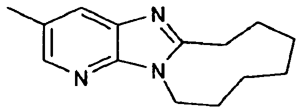

Method A applied to 2-Chloro-5-methyl-3-nitropyridine (86 mg, 0.5 mmol) and 2-azacyclononanone (85 mg, 0.6 mmol) afforded the title compound as a viscous oil (40 mg, 35%). ¹H NMR (DMSO) δ 1.18-1.96 (m, 10 H), 2.48 (s, 3 H), 3.18 (t, *J* = 6.0 Hz, 2 H), 4.54 (t, *J* = 6.5 Hz, 2 H), 7.99 (s, 1 H), 8.36 (s, 1 H).

### Example 15: 3-Methyl-8,8-diphenyl-6,7,8,9-tetrahydro-dipyrido[1,2-a;3',2'-d]imidazole.

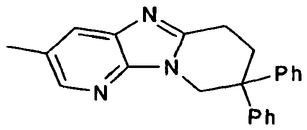

Method A applied to 2-Chloro-5-methyl-3-nitropyridine (86 mg, 0.5 mmol) and 5,5-diphenyl-piperidin-2-one (151 mg, 0.6 mmol) afforded the title compound as solid (93 mg, 55%). ¹H NMR (DMSO) δ 2.48-2.54 (m, 4 H), 2.93 (s, 3 H), 4.87 (s, 2 H), 7.18-7.34 (m, 10 H), 7.98 (s, 1 H), 8.41 (s, 1 H).

### Example 16:

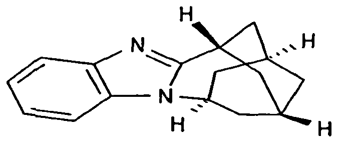

Method A applied to 1,2-iodonitrobenzene (125 mg, 0.5 mmol) and (1*R*,3*R*,6*S*,8*S*)-4-Aza-tricyclo[4.3.1.1*3,8*]undecan-5-one (99 mg, 0.6 mmol) afforded the title compound as a solid (99 mg, 83%). ¹H NMR (DMSO) δ 1.84-2.23 (m, 12 H), 3.54-3.57 (m, 1 H), 5.17 (s, 1 H), 7.53-7.58 (m, 2 H), 7.80 (d, *J* = 7.2 Hz, 1 H), 7.98 (d, *J* = 7.2 Hz, 1H).

### Example 17: 2,3-Dihydro-1H-benzo[d]pyrrolo[1,2-a]imidazole.

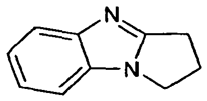

A reaction tube containing 2-iodonitrobenzene (125 mg, 0.5 mmol), pyrrofidin-2-one (51 mg, 0.6 mmol), CuI (4.8 mg, 0.025 mmol), *N*-methylethylenediamine (4.4 µL, 0.05 mmol), potassium phosphate (212 mg, 1 mmol) in dry toluene (3 mL) was purged with dry argon for 3 min. Then the mixture was heated at 100°C for 18 h. (In other reactions *trans*-1,2-cyclohexanodiamine was used instead of *N-*methylethylenediamine). After cooling, the reaction was hydrolyzed with 3 mL of water and filtered through a Varian cartridge Chem Elut 12198007, rinsing with ethyl acetate. The crude mixture was dissolved in 10 mL of glacial acetic acid and refluxed for 30 min in the presence of iron powder (279 mg, 5 mmol). The acid was removed under reduced pressure and the residue was suspended in saturated sodium bicarbonate solution and extracted with ethyl acetate. The obtained crude was purified by preparative HPLC, affording the title compound as a colorless solid (58 mg, 73%). mp 86-88°C. ¹H NMR δ 2.75 (t, *J* = 6.9 Hz, 2 H), 3.24-3.33 (m, 2 H), 4.33 (t, *J* = 7.2 Hz, 2 H), 7.46-7.50 (m, 2 H), 7.74-7.86 (m, 2 H); ¹³C NMR δ 23.7, 25.3, 45.3, 112.7, 115.1, 124.7, 125.0, 128.9, 136.6, 157.0. HRMS (FAB): cal. for C₁₀H₁₁N₂ [M+H⁺]: 159.0922; found: 159.0919.

### Example 18: 7-methyl-2,3-dihydro-1H-benzo[d]pyrrolo[1,2-a]imidazole

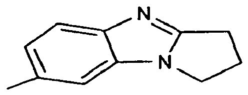

The same method was applied to 2-Chloro-4-methyl-1-nitrobenzene (86 mg, 0.5 mmol) and pyrrolidin-2-one (51 mg, 0.6 mmol), using *N*-methylethylenediamine as ligand (4.4 µL, 0.05 mmol) and afforded the title compound as a viscous oil (72 mg, 84%). ¹H NMR (DMSO) δ 2.73-2.82 (m, 2 H), 3.32 (t, *J* = 7.1 Hz), 4.35 (t, *J* = 7.2 Hz, 2 H), 7.33 (d, *J* = 8.3 Hz, 1 H), 7.57 (s, 1 H), 7.69 (d, *J* = 8.3 Hz, 1 H); ¹³C NMR δ 21.0, 23.8, 25.3, 45.6, 112.5, 114.3, 126.4, 127.1, 135.5, 136.1, 157.8.

### Example 19: 6-methyl-2,3-dihydro-1H-benzo[d]pyrrolo[1,2-a]imidazole

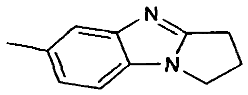

The same method was applied to 1-Chloro-4-methyl-2-nitrobenzene (86 mg, 0.5 mmol) and pyrrolidin-2-one (51 mg, 0.6 mmol), using *N*-methylethylenediamine (4.4 µL, 0.05 mmol) as ligand, and afforded the title compound as a viscous oil (74 mg, 86%). ¹H NMR (DMSO) δ 2.55-2.63 (m, 2 H), 2.76 (s, 3 H), 3.21 (t, *J* = 7.7 Hz, 2 H), 3.85 (s, 3 H), 4.26 (t, *J* = 7.1 Hz, 2 H), 7.56 (d, *J* = 8.6 Hz, 1 H), 7.85 (d, *J* = 8.6 Hz, 1 H); ¹³C NMR δ 21.0, 23.7, 25.2, 45.3, 112.4, 114.5, 126.5, 129.2, 134.8, 137.8, 157.2.

### Example 20: 7-methoxy-2,3-dihydro-1H-benzo[d]pyrrolo[1,2-a]imidazole.

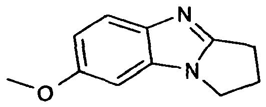

The same method was applied to 1-Iodo-4-methoxy-2-nitrobenzene (140 mg, 0.5 mmol) and pyrrolidin-2-one (51 mg, 0.6 mmol), using *N*-methylethylenediamine (4.4 µL, 0.05 mmol) as ligand, and afforded the title compound as a viscous oil (84 mg, 89%). ¹H NMR (DMSO) δ 2.74 (t, *J* = 6.9 Hz, 2 H), 3.24-3.32 (m, 2 H), 3.86 (s, 3 H), 4.32 (t, *J* = 7.1 Hz, 2 H), 7.09 (d, *J* = 9.1 Hz, 1 H), 7.44 (d, *J* = 9.1 Hz, 1 H), 7.58 (s, 1 H); ¹³C NMR δ 23.6, 25.3, 45.4, 55.9, 96.0, 114.6, 115.6, 128.7, 129.6, 157.4, 158.2.

### Example 21: 5-Methyl-2,3-dihydro-1H-benzo[d]pyrrolo[1,2-a]imidazole-6-carboxylic acid methyl ester.

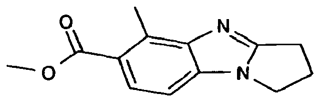

The same method was applied to 4-Bromo-2-methyl-3-nitrobenzoic acid methyl ester (137 mg, 0.5 mmol) and pyrrolidin-2-one (51 mg, 0.6 mmol), using *N*-methylethylenediamine (4.4 µL, 0.05 mmol) as ligand, and afforded the title compound as a viscous oil (89 mg, 77%). ¹H NMR (DMSO) δ 2.55-2.63 (m, 2 H), 2.76 (s, 3 H), 3.21 (t, *J* = 7.7 Hz, 2 H), 3.85 (s, 3 H), 4.26 (t, *J* = 7.1 Hz, 2 H), 7.56 (d, *J* = 8.6 Hz, 1 H), 7.85 (d, *J* = 8.6 Hz, 1 H); ¹³C NMR 6 14.7, 23.4, 25.4, 44.5, 51.9, 99.1, 109.0, 124.0, 125.6, 129.2, 131.6, 161.6, 167.1.

### Example 22: 2-Methoxy-7,8-dihydro-6H-pyrrolo[2',1':2,3]imidazo[4,5-b]pyridine

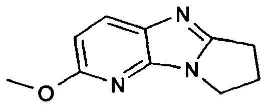

The same method was applied to 2-Chloro-6-methoxy-3-nitropyridine (94 mg, 0.5 mmol) and pyrrolidin-2-one (51 mg, 0.6 mmol), using *trans*-1,2-cyclohexanodiamine as ligand (6 µL, 0.05 mmol), and afforded the title compound as a viscous oil (28 mg, 30%). ¹H NMR (DMSO) 6 2.68 (p, *J* = 6.9 Hz, 2 H), 3.23 (t, *J* = 6.9 Hz, 2 H), 3.91 (s, 3 H), 4.22 (t, *J* = 6.9 Hz), 6.82 (d, *J* = 8.6 Hz, 1 H), 8.02 (d, *J* = 8.6 Hz, 1 H).

### Example 23: 2,6-Dimethyl-7,8-dihydro-6H-pyrrolo[2',1':2,3]imidazo[4,5-b]pyridine

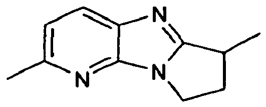

The same method was applied to 2-Bromo-6-methyl-3-nitropyridine (109 mg, 0.5 mmol) and 3-methylpyrrolidin-2-one (59 mg, 0.6 mmol), using *trans*-1,2-cyclohexanodiamine as ligand (6 µL, 0.05 mmol), and afforded the title compound as a viscous oil (77 mg, 83%). ¹H NMR (DMSO) δ 1.42 (d, *J* = 6.9 Hz, 3 H), 2.22-2.32 (m, 1 H), 2.55 (s, 3 H), 2.86-2.97 (m, 1 H), 3.53-2.61 (m, 1 H), 4.12-4.38 (m, 2 H), 7.27 (d, *J* = 8.0 Hz, 1 H), 7.99 (d, *J* = 8.0 Hz, 1 H).

### Example 24:

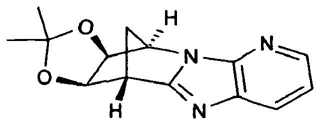

The same method was applied to 2-Bromo-3-nitropyridine (101 mg, 0.5 mmol) and (1*S*,2*R*,6*S*,7*R*)-4,4-dimethyl-3,5-dioxa-8-azatricyclo[5.2.1.0*2,6*]decan-9-one (110 mg, 0.6 mmol), using *trans*-1,2-cyclohexanodiamine as ligand (6 µL, 0.05 mmol), and afforded the title compound as a viscous oil (57 mg, 44%). ¹H NMR (DMSO) δ 1.23 (s, 3 H), 1.48 (s, 3 H), 2.47-2.57 (m, 2 H), 2.53 (s, 3 H), 3.64 (s, 3 H), 4.22 (d, *J* = 4.8 Hz, 1 H). 4.34 (d, *J* = 4.8 Hz, 1 H), 7.22 (dd, *J* = 7.9, 5.2 Hz, 1 H), 7.99 (d, *J* = 7.9 Hz, 1 H), 8.27 (d, *J* = 5.2 Hz, 1 H).

### Example 25: 1,2,3,4-Tetrahydro-benzo[4,5]imidazo[1,2-a]pyridine

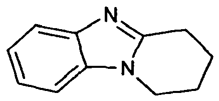

The same method was applied to 1-lodo-2-nitrobenzene (125 mg, 0.5 mmol) and piperidin-2-one (59 mg, 0.6 mmol), using *N*-methylethylenediamine (4.4 µL, 0.05 mmol) as ligand, and afforded the title compound as pale yellow solid (65 mg, 75%). mp 104-106°C.¹H NMR (DMSO) δ 1.98-2.07 (m, 4 H), 3.16-3.23 (m, 2 H), 4.30 (t, *J* = 6.9 Hz, 2 H), 7.51-7.56 (m, 2 H), 7.75-7.92 (m, 2 H); ¹³C NMR δ 17.7, 20.5, 22.3, 43.0, 112.3, 114.2, 124.7, 125.6, 131.5, 151.8, 156.6. HRMS (FAB): cal. for C₁₁H₁₃N₂ [M+H⁺]: 173.1079; found: 173.1071.

### Example 26: 2-Methyl-8,8-diphenyl-6,7,8,9-tetrahydro-dipyrido[1,2-a:3',2'-d]imidazole

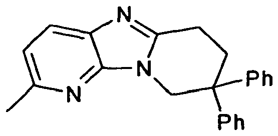

The same method was applied to 2-Bromo-6-methyl-3-nitropyridine (86 mg, 0.5 mmol) and 5,5-diphenylpiperidin-2-one (151 mg, 0.6 mmol), using *trans*-1,2-cyclohexanodiamine as ligand (6 µL, 0.05 mmol), and afforded the title compound as solid (87 mg, 51%). ¹H NMR (DMSO) δ 2.68 (s, 3 H), 2.88-2.97 (m, 4 H), 4.82 (s, 2 H), 7.21-7.33 (m, 10 H), 7.38 (d, *J* = 8.2 Hz, 1 H), 8.04 (d, *J* = 8.2 Hz, 1 H).

### Example 27: 5,6,7,8,9,10-Hexahydro-1,4b, 11-triaza-cyclooct[a]indene

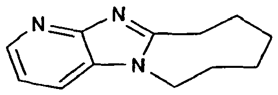

The same method was applied to 3-Bromo-2-nitropyridine (102 mg, 0.5 mmol) and 2-azacyclooctanone (76 mg, 0.6 mmol), using *trans-*1,2-cyclohexanodiamine as ligand (6 µL, 0.05 mmol), and afforded the title compound as a viscous oil (20 mg, 20%). ¹H NMR (DMSO) δ 1.16-1.25 (m, 2 H), 1.41-1.52 (m, 2 H), 1.82-1.91 (m, 4 H), 3.26 (t, *J* = 6.2 Hz, 2 H), 4.61 (t, *J* = 6.1 Hz, 2 H), 7.59 (dd, *J* = 8.2, 5.5 Hz, 1 H), 8.48 (d, *J* = 8.2 Hz, 1 H), 8.62 (d, *J* = 5.5 Hz, 1 H).

### Example 28: 3-Methoxy-6,7,8,9,10,11-hexahydro-5H-4b,12-diaza-cyclonon[a]indene.

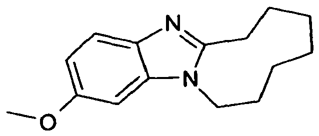

The same method was applied to 3-lodo-4-nitroanisole (140 mg, 0.5 mmol) and 2-azacyclononanone (85 mg, 0.6 mmol), using *N*-methylethylenediamine (4.4 µL, 0.05 mmol) as ligand, and afforded the title compound as brown solid (70 mg, 57%). ¹H NMR (DMSO) δ 1.18-1.96 (m, 10 H), 3.23 (t, *J* = 6.4 Hz, 2 H), 3.88 (s, 3 H), 4.61 (t, *J* = 6.1 Hz, 2 H), 7.17 (dd, *J* = 8.6, 3.1 Hz, 1 H), 7.52 (d, *J* = 3.1 Hz, 1 H), 7.73 (d, *J* = 8.6 Hz, 1 H).

## Claims

1. A process for preparing a compound of formula I 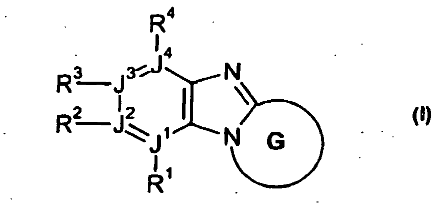 and/or all stereoisomeric forms of the compound of formula I, and/or mixtures of these forms in any ratio, and/or a physiologically tolerated salt of the compound of formula I, wherein
J1, J2, J3 and J4 are independently from each other selected from carbon or nitrogen atoms and form together with the carbon atoms they are attached to a stable aromatic or heteroaromatic ring,
G is selected from azetidine, azepane, azocane, aza-bicyclo[2.2.1]heptane, aza-bicyclo[2.2.2]octane, azacyclooctanone, azacyclononanone, aza-tricyclo [4.3.1.1*3,8*]undecane, 4,4-dimethyl-3,5-dioxa-azatricyclo[5.2.1.0*2,6*]-decane, 3,5-dioxa-azatricyclo-[5.2.1.0*2,6*]decane, 4,4-dimethyl-3,5-dioxa-azatricyclo [5.2.1.0*2,6*]decan-9-one. azocane-2-one, azonane, 1,4-diazepane, [1,4]diazocane, [1,2]diazocan-3-one, [1,3]diazocan-2-one, imidazoline, imidazolidine, isothiazolidine, isoxazolidine, ketopiperazine, morpholine, [1,4]oxazocane, [1,3]oxazocan-2-one, piperazine, piperidine, pyrazoline, pyrazolidine, 1,2-dihydro-pyridine, pyrrolidine, pyrrolidinone, 2,3-dihydro-1H-pyrrole, pyrroline, 5,6,7,8-tetrahydro-1H-azocin-2-one, tetrahydropyridine, thiadiazine, thiazolidine, thiazoline or thiomorpholine,
wherein G is unsubstituted or mono-, di-, tri- or four times substituted independently of one another by oxo or by R5,
R1, R2, R3, R4 and R5 are independent of one another identical or different and are
a) hydrogen atom,
b) -(C₁-C₄)₋alkyl, wherein alkyl is unsubstituted or substituted one to three times by R13,
c) halogen,
d) phenyloxy-, wherein phenyloxy is unsubstituted or substituted one to three times by R13,
e) -(C₁-C₃)-fluoroalkyl,
f) -N(R10)-(C₁-C₄)-alkyl, wherein alkyl is unsubstituted or substituted one to three times by R13,
g) -(C₆-C₁₄)-aryl, wherein aryl is unsubstituted or mono-, di-, tri- or four times substituted independently of one another by R13,
h) -(C₄-C₁₄)-heteroaryl, wherein heteroaryl is unsubstituted or mono-, di-, tri- or four times substituted independently of one another by R13,
i) -(C₃-C₈)-cycloalkyl, wherein said cycloalkyl is unsubstituted or mono-, di- , tri- or four times substituted independently of one another by R13,
j) a 3- to 7-membered cyclic residue, containing 1, 2, 3 or 4 heteroatoms chosen from nitrogen, sulfur or oxygen, wherein said cyclic residue is unsubstituted or mono-, di-, tri- or four times substituted independently of one another by R13,
k) -O-CF₃,
l) -O-(C₁-C₄)-alkyl, wherein alkyl is unsubstituted or substituted one to three times by R13,
m) -NO₂,
n) -CN,
o) -OH,
p) -C(O)-R10,
q) -C(O)-O-R11,
r) -C(O)-N(R11)-R12,
s) -N(R11)-R12,
t) -N(R10)-SO₂-R10,
v) -S-R10,
w) -SOₙ-R10, wherein n is 1 or 2,
x) -SO₂-N(R11)-R12 or
y) at least one of R1, R2, R3 or R4 are absent in case one or more of J1, J2, J3 or J4 are nitrogen atom, or
R1 and R2, R2 and R3 or R3 and R4 form together with the atoms which they are attached to a 5- or 8- membered ring, containing up to 0, 1, 2, 3 or 4 heteroatoms chosen from nitrogen, sulfur or oxygen, wherein said ring is unsubstituted or substituted one, two, three or four times by R14,
R10 is hydrogen atom, -(C₁-C₃)-fluoroalkyl or -(C₁-C₆)-alkyl,
R11 and R12 are independently of one another identical or different and are
a) hydrogen atom,
b) -(C₁-C₆)-alkyl, wherein alkyl is unsubstituted or mono-, di- or trisubstituted independently of one another by R13,
c) -(C₆-C₁₄)-aryl-, wherein aryl is unsubstituted or mono-, di- or trisubstituted independently of one another by R13,
d) -(C₄-C₁₄)-heteroaryl, wherein heteroaryl is unsubstituted or mono-, di- or trisubstituted independently of one another by R13 or
R13 is halogen, -NO₂, -CN, =O, -OH, -(C₁-C₈)-alkyl, -(C₁-C₈)-alkoxy , -CF₃, phenyloxy-, -C(O)-R10, -C(O)-O-R17, -C(O)-N(R17)-R18, -N(R17)-R18, -N(R10)-SO2-R10, -S-R10, -SOₙ-R10, wherein n is 1 or 2, -SO₂-N(R17)-R18, -(C₆-C₁₄)-aryl, wherein aryl is unsubstituted or mono-, di- or trisubstituted independently of one another by R14, -(C₄-C₁₄)-heteroaryl, wherein heteroaryl is unsubstituted or mono-, di- or trisubstituted independently of one another by R14, -(C₃-C₈)-cycloalkyl, wherein said cycloalkyl is unsubstituted or mono-, di- or trisubstituted independently of one another by R14, or a 3- to 7-membered cyclic residue, containing 1, 2, 3 or 4 heteroatoms chosen from nitrogen, sulfur or oxygen, wherein said cyclic residue is unsubstituted or mono-, di- or trisubstituted independently of one another by R14,
R14 is halogen, -OH, =O, -CN, -CF₃, -(C₁-C₈)-alkyl, -(C₁-C₄)-alkoxy, -NO₂, -C(O)-OH, -NH₂, -C(O)-O-(C₁-C₄)-alkyl, -(C₁-C₈)-alkylsulfonyl, -C(O)-NH-(C₁-C₈)-alkyl, -C(O)-N-[(C₁-C₈)-alkyl]₂, -C(O)-NH₂, -S-R10, -N(R10)-C(O)-NH-(C₁-C₈)-alkyl, or -N(R10)-C(O)-N-[(C₁-C₈)-alkyl]₂,
R17 and R18 are independently of one another identical or different and are
a) hydrogen atom,
b) -(C₁-C₆)-alkyl,
c) -(C₆-C₁₄)-aryl- or
d) -(C₄-C₁₄)-heteroaryl,
said process comprises a reaction of a compound of formula II 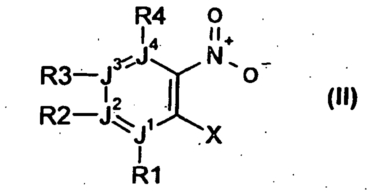 wherein R1, R2, R3, R4, J1, J2, J3 and J4 are as defined in formula I and
X is Cl, Br, I, triflate or nonaflate, with a compound of formula III 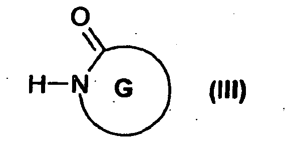 wherein ring G are as defined in formula I,
in the presence of a metal catalyst, selected from palladium and copper, a base, a ligand, wherein the ligand is selected out of the group (+/-)-2,2'-bis(diphenylphosphino)-1,1'-binaphthalene, (*R*)-(-)-1-[(*S*)-2-(diphenylphosphino) ferrocenyl] ethyldicyclohexylphosphine, 1,2-Bis(diphenylphosphino)ethane , 1,3-Bis(diphenylphosphino)propane, (*R*)-(-)-1-[(*S*)-2-(Dicyclohexylphosphino)-ferrocenyl]ethyldi-tert-butylphosphine, (*R*)-(+)-1,1'-Bis(diphenylphosphino)-2,2'-bis(*N,N*-diiisopropylamido)ferrocene, (*S*,*S*)-1-[1-(Di-*tert*-butylphosphino)ethyl]-2-(diphenylphosphino)ferrocene, (1*R*,2*R*)-(+)-1,2-Diaminocyclohexane-*N,N'*-bis(2-diphenylphosphino-1-naphtoyl, (-)-1,2-Bis((2*S*,5*S*)-2,5-diisopropylphospholano)-benzene, Bis[(2-diphenylphosphino)phenyl]ether, (*S*)-(-)-2,2'-Bis(di-para-tolylphosphino)-1,1'-binaphyl, 4,5-Bis(bis(3,5-bis(trifluoromethyl)phenyl)-phosphino)-9,9-dimethylxanthen, 2,2'-bis[(2',4',6'-triisopropyl)dicyclohexyl-phosphino]biphenyl and 2,2'-bis(di-*tert-*butylphosphino)biphenyl, tri-*tert*-butylphosphine, if the metal catalyst is palladium or
the ligand is selected out of the group ethylenediamine, *N*-methylethylenediamine, *N,N*-dimethyl-ethane-1,2-diamine *N*-buthylethylenediamine, *N,N-*dimethylethylenediamine, *N*,*N*,*N*'-trimethylthylenediamine, *N,N,N,N'-*tetramethylthylenediamine, *trans*-1,2-cyclohexanodiamine, *cis*-1,2-cyclohexanodiamine, *cis*/*trans*-1,2-cyclohexanodiamine, *N*,*N*'-diethyl-1,2-cyclohexanodiamine, *N*,*N*'-dipropyl-1,2-cyclohexanodiamine, 1,3-propylenediamine, 1,2-benzenediamine, phenanthridine, acridine, acridine orange, 9-aminoacridine, 9-hydroxy-4-methoxyacridine, proflavine, 4-(2-pyriylazo) resorcinol, 1,2-dihydro-1-(2-(2-pyridyl)-ethyl)-3,6-pyridazinedione; [1,10]phenanthroline, 5-nitro-[1,10]phenanthroline, bathophenanthroline, spiramycin, bicinchonic acid sodium salt (bca), 1-(4-pyridyl) pyridinium chloride, 2-pyridylacetic acid hydrochloride, 8-mercaptoquinoline hydrochloride, dimethylamino acetic acid, picolinic acid, 3-hydroxypicolinic acid, 3-hydroxy picolinamide, glycol, pyridine, 2-aminopyridine, 2-hydroxipyridine, 3-cyanopyridine, 4-cyanopyridine, 2-ethylpyridine, 2-amino-6-methylpyridine, 2-(aminomethylpyridine), 2-(hydroximethylpyridine), 2-hydroxi-6-methylpyridine, 2-dimethylaminopyridine, 4-dymethylaminopyridine, 2-(2-hydroxiethyl)pyridine, 4-tert-butylpyridine, 3-acetoxypyridine, 2-phenylpyridine, 4-phenylpyridine, 4-benzoylpyridine, 2-(2-thienyl)pyridine, 2-benzylpyridine, 2-anilinopyridine, 3-pyridinepropanol, 1-(2-pyridyl) piperazine, di-2-pyridyl ketone, ethyl 2-pyridyl acetate, 2-(2-diethylaminoethyl)-pyridine, 4-(2-diethylaminoethyl)pyridine, 2,6-di-tert-butyl pyridine, (*S*,*S*)-2,6-bis(4-isopropyl-2-oxazolin-2-yl) pyridine, 2,3-pyridine dicarboxylic acid, 2,6-pyridine dicarboxylic acid, 3,5-pyridine dicarboxylic acid, 1,3-di(4-pyridyl)propane, 2,3-di-3-pyridyl-2,3-butanediol, 2,2'-bipyridine, 2,2-dipyridyl, 4,4'-dimethyl-2,2'-dipyridyl, 3-hydroxypyridine, 2-mercaptopyridine, 2-(2-methylaminoethyl) pyridine, 3-hydroxi picolinamine, 3-hydroxypicolinic acid, 2,2':6',2"-terpyridine, 2-picoline, 6,6'-bi-2-picoline, 2,4-lutidine, 2,6-lutidine-α-2,3-diol, 2,6-lutidine2,4,6-collidine, picolinamide, ethyl picolinate, ethyl isonicotinate, quinoline, 2-phenylquinoline, 8-hidroxyquinoline, 8-acetoxyquinoline, 2-methyl-8-nitroquinoline, 7,8-benzoquinoline, 2-quinolinol, 2-quinolinethiol, quinoline-4-carboxylic acid, 2-phenyl-4-quinoline carboxylic acid, 2,4-hydroxy quinoline monosodium salt, 8-ethoxyquinoline-5-sulfonic acid sodium salt, 8-hydroxy-5-nitroquinoline, 4-chloro-7-(trifluoromethyl) quinoline, 8-hydroxyquinoline-5-sulfonic acid monohydrate, 5-nitroquinaldic acid, isoquinoline, isoquinoline-3-carboxylic acid hydrate, 1,4,5-triazanaphtalene, quinaldine, 4-chloroquinaldine, nicotine, isonicotinamine, neocuproine, lysine, cystine, α-alanine, arginine, cysteine and β-alanine, if the metal catalyst is copper
and an aprotic solvent to give a compound of formula IV 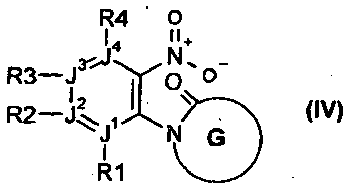 and converting the compound of formula IV into a compound of formula I in the presence of a reducing reagent and a second solvent and
optionally the compound of formula I is converted to its physiologically tolerated salt.

2. The process according to claim 1, wherein a compound of formula I is prepared,
wherein
J1, J2, J3 and J4 form together with the carbon atoms they are attached to a ring selected from benzene, pyrazine, pyridazine, pyridine, pyrimidine, triazine or tetrazine,
G is selected from azetidine, azepane, azocane, aza-bicyclo[2.2.1]heptane, aza-bicyclo[2.2.2]octane, azacyclooctanone, azacyclononanone, aza-tricyclo [4.3.1.1*3,8*]undecane, 4,4-dimethyl-3,5-dioxa-azatricyclo[5.2.1.0*2,6*]-decane, 3,5-dioxa-azatricyclo-[5.2.1.0*2,6*]decane, 4,4-dimethyl-3,5-dioxa-azatricyclo [5.2.1.0*2,6*]decan-9-one, azocane-2-one, azonane, 1,4-diazepane, [1,4]diazocane, [1,2]diazocan-3-one, [1,3]diazocan-2-one, imidazoline, imidazolidine, isothiazolidine, isoxazolidine, ketopiperazine, morpholine, [1,4]oxazocane, [1,3]oxazocan-2-one, piperazine, piperidine, pyrazoline, pyrazolidine, 1,2-dihydro-pyridine, pyrrolidine, pyrrolidinone, 2,3-dihydro-1H-pyrrole, pyrroline, 5,6,7,8-tetrahydro-1H-azocin-2-one, tetrahydropyridine, thiadiazine, thiazolidine, thiazoline or thiomorpholine,
wherein G is unsubstituted or mono-, di-, tri- or four times substituted independently of one another by oxo or by R5,
R1, R2, R3, R4 and R5 are independent of one another identical or different and are
a) hydrogen atom,
b) F,
c) Cl,
d) -(C₁-C₄)-alkyl, wherein alkyl is unsubstituted or substituted one to three times by R 13,
e) -(C₁-C₃)-fluoroalkyl,
f) phenyl, wherein phenyl is unsubstituted or substituted one to three times by R13,
g) -(C₄-C₁₄)-heteroaryl, wherein heteroaryl is selected from acridinyl, azaindole (1 H-pyrrolopyridinyl), azabenzimidazolyl; azaspirodecanyl, azepinyl, azetidinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, carbazolyl, 4aH-carbazolyl, carbolinyl, chromanyl, chromenyl, cinnolinyl, decahydrochinolinyl, 4,5-dihydrooxazolinyl, dioxazolyl, dioxazinyl, 1,3-dioxolanyl, 1,3-dioxolenyl, 3,3-dioxo[1,3,4]oxathiazinyl, 6H-1,5,2-dithiazinyl, dihydrofuro[2,3-b]-tetrahydrofuranyl, furanyl, furazanyl, imidazolidinyl, imidazolinyl, imidazolyl, indanyl, 1 H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isothiazolidinyl, isothiazolinyl, isoxazolyl, isoxazolinyl, isoxazolidinyl, 2-isoxazolinyl, ketopiperazinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, 1,2-oxa-thiepanyl, 1,2-oxathiolanyl, 1,4-oxazepanyl, 1,4-oxazepinyl, 1,2-oxazinyl, 1,3-oxazinyl, 1,4-oxazinyl, oxazolidinyl, oxazolinyl, oxazolyl, oxetanyl, oxocanyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyrazolo[3,4-b]pyridine, pyridazinyl, pyridooxazolyl, pyridoimidazolyl, pyridothiazolyl, pyridinyl, pyridyl, pyrimidinyl, pyrrolidinyl, pyrrolidinonyl, pyrrolinyl, 2H-pyrrolyl, pyrrolyl, quinazolinyl, quinolinyl, 4H-quinolizinyl, quinoxalinyl, quinuclidinyl, tetrahydrofuranyl, tetrahydroisoquinolinyl, tetrahydroquinolinyl, tetrahydrofuranyl, tetrahydropyranyl, tetrahydropyridinyl, tetrahydrothiophenyl, tetrazinyl, tetrazolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, 1,2-thiazinyl, 1,3-thiazinyl, 1,4-thiazinyl, 1,3-thiazolyl, thiazolyl, thiazolidinyl, thiazolinyl, thienyl, thietanyl, thienothiazolyl, thienooxazolyl, thienoimidazolyl, thietanyl, thiomorpholinyl, thiophenolyl, thiophenyl, thiopyranyl, 1,2,3-triazinyl, 1,2,4-triazinyl, 1,3,5-triazinyl, 1,2,3-triazolyl, 1,2,3-triazolyl, 1,2,4-triazolyl, 1,2,5-triazolyl, 1,3,4-triazolyl and xanthenyl" and is unsubstituted or mono-, di-, tri- or four times substituted independently of one another by R13,
h) -(C₃-C₈)-cycloalkyl, wherein said cycloalkyl is unsubstituted or mono-, di-, tri- or four times substituted independently of one another by R13, or
i) a 3- to 7-membered cyclic residue is selected from azepine, azetidine, aziridine, azirine, 1,4 diazepane, 1,2-diazepine, 1,3-diazepine, 1,4-diazepine, diaziridine, diazirine, dioxazole, dioxazine, dioxole, 1,3-dioxolene, 1,3-dioxolane, furan, imidazole, imidazoline, imidazolidine, isothiazole, isothiazolidine, isothiazoline, isoxazole, isoxazoline, isoxazolidine, 2-isoxazoline, ketomorpholine, ketopiperazine, morpholine, 1,2-oxa-thiepane, 1,2-oxathiolane, 1,4-oxazepane, 1,2-oxazine, 1,3-oxazine, 1,4-oxazine, oxazole, oxaziridine, oxetan, oxirane, piperazine, piperidine, pyran, pyrazine, pyrazole, pyrazoline, pyrazolidine, pyridazine, pyridine, pyrimidine, pyrrole, pyrrolidine, pyrrolidinone, pyrroline, tetrahydrofuran, tetrahydropyran, tetrahydropyridine, tetrazine, tetrazole, thiadiazine thiadiazole, 1,2-thiazine, 1,3-thiazine, 1,4-thiazine, 1,3-thiazole, thiazole, thiazolidine, thiazoline, thienyl, thietan, thiomorpholine, thiopyran, 1,2,3-triazine, 1,2,4-triazine, 1,3,5-triazine, 1,2,3-triazole or 1,2,4-triazole, and is unsubstituted or mono-, di-, tri- or four times substituted independently of one another by R13,
j) -O-CF3,
k) -O-(C₁-C₄)-alkyl, wherein alkyl is unsubstituted or substituted one to three times by R13,
l) -N(R10)-(C₁-C₄)-alkyl, wherein alkyl is unsubstituted or substituted one to three times by R13,
m) -CN,
n) -OH,
o) phenyloxy-, wherein phenyloxy is unsubstituted or substituted one to three times by R13,
p) -C(O)-O-R11,
q) -C(O)-N(R11)-R12,
r) -N(R11)-R12,
s) -N(R10)-SO2-R10,
t) -S-R10,
v) -SOₙ-R10, wherein n is 1 or 2,
w) -SO₂-N(R11)-R12,
x) -C(O)-R10 or
y) at least one of R1, R2, R3 or R4 are absent in case one or more of J1, J2, J3 or J4 are nitrogen atom,
R10 is hydrogen atom, -(C₁-C₃)-fluoroalkyl or-(C₁-C₆)-alkyl,
R11 and R12 are independently of one another identical or different and are
a) hydrogen atom,
b) -(C₁-C₄)-alkyl, wherein alkyl is unsubstituted or mono-, di- or trisubstituted independently of one another by R13,
c) phenyl, wherein phenyl is unsubstituted or mono-, di- or trisubstituted independently of one another by R13,
d) -(C₄-C₁₄)-heteroaryl, wherein heteroaryl is as defined above and is unsubstituted or mono-, di- or trisubstituted independently of one another by R13 or
R13 is F, Cl, -CN, =O, -OH, -(C₁-C₈)-alkyl, -(C₁-C₈)-alkoxy, -CF₃, phenyloxy-, -C(O)-R10, -C(O)-O-R17, -C(O)-N(R17)-R18, -N(R17)-R18, -N(R10)-SO₂-R10, -S-R10, -SOₙ-R10, wherein n is 1 or 2, -SO₂-N(R17)-R18, phenyl, wherein phenyl is unsubstituted or mono-, di- or trisubstituted independently of one another by R14, -(C₄-C₁₄)-heteroaryl, wherein heteroaryl is as defined above and is unsubstituted or mono-, di- or trisubstituted independently of one another by R14, -(C₃-C₆)-cycloalkyl, wherein said cycloalkyl is unsubstituted or mono-, di- or trisubstituted independently of one another by R14, or a 3- to 7-membered cyclic residue, which is as defined above and is unsubstituted or mono-, di- or trisubstituted independently of one another by R14,
R14 is F, Cl, -OH, =O, -CN, -CF₃, -(C₁-C₈)-alkyl, -(C₁-C₄)-alkoxy, -C(O)-OH, -NH₂, -C(O)-O-(C₁-C₄)-alkyl, -(C₁-C₈)-alkylsulfonyl, -C(O)-NH₂, -C(O)-NH-(C₁-C₈)-alkyl, -C(O)-N-[(C₁-C₈)-alkyl]₂, -S-R10, -N(R10)-C(O)-NH-(C₁-C₈)-alkyl or -N(R10)-C(O)-N-[(C₁-C₈)-alkyl]₂, R17 and R18 are independently of one another identical or different and are
a) hydrogen atom,
b) -(C₁-C₄)-alkyl,
c) phenyl or
d) -(C₄-C₁₄)-heteroaryl, wherein heteroaryl is as defined above and
X is Cl, Br or I.

3. The process according to claim 1 wherein one of the following compounds of formula I is prepared:
2,3-Dihydro-1*H*-benzo[d]pyrrolo[1,2-a]imidazole;
7-Methyl-2,3-dihydro-1*H*-benzo[d]pyrrolo[1,2-a]imidazole;
6-Methyl-2,3-dihyoro-1*H*-benzo[d]pyrrolo[1,2-a]imidazole;
7-Methoxy-2,3-dihydro-1*H*-benzo[d]pyrrolo[1,2-a]imidazole;
5-Methyl-2,3-dihydro-1*H*-benzo[d]pyrrolo[1,2-a]imidazole-6-carboxylic acid methyl ester;
2-Methoxy-7,8-dihydro-6*H*-pyrrolo[2',1':2,3]imidazo[4,5-b]pyridine;
2,6-Dimethyl-7,8-dihydro-6*H*-pyrrolo[2',1':2,3]imidazo[4,5-b]pyridine; 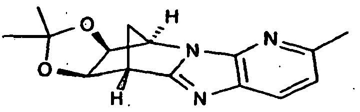
1,2,3,4-Tetrahydro-benzo[4,5]imidazo[1,2-a]pyridine;
3,9-Dimethyl-6,7,8,9-tetrahydro-dipyrido[1,2-a;3',2'-d]imidazole;
7-Chloro-4,4-diphenyl-1,2,3,4-tetrahydro-benzo[4,5]imidazo[1,2-a]pyridine;
Dimethyl-(*S*)-7,8,9,10-tetrahydro-6*H*-benzo[4,5]imidazo[1,2-a]azepin-6-yl-amine;
3-Methyl-5,6,7,8,9,10-hexahydro-4,4b,11-triaza-cycloocta[a]indene;
2-Methyl-6,7,8,9,10,11-hexahydro-5*H*-4,4b,12-triaza-cyclonona[a]indene;
3-Methyl-8,8-diphenyl-6,7,8,9-tetrahydro-dipyrido[1,2-a;3',2'-d]imidazole; 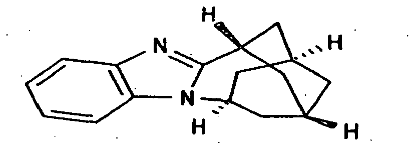
2-Methyl-8,8-diphenyl-6,7,8,9-tetrahydro-dipyrido[1,2-a:3',2'-d]imidazole;
5,6,7,8,9,10-Hexahydro-1,4b,11-triaza-cyclooct[a]indene; or
3-Methoxy-6,7,8,9,10,11-hexahydro-5*H*-4b,12-diaza-cyclonon[a]indene.

4. The process according to claim 1, wherein the palladium catalyst is selected from: Pd-alkanoates, Pd-alkanoate complexes, Pd-acetonates; Pd-halides, Pd-halide complexes and Pd-phosphine complexes.

5. The process according to claim 4, wherein the palladium catalyst is selected from: palladium (II) acetate, palladium (II) trifluoroacetate, tris(dibenzylideneacetone)dipalladium(0), tris(dibenzylideneacetone)dipalladium(0) chloroform adduct, palladium (II) chloride, 2,2'-bis(diphenylphosphino)-1,1'-binaphthylpalladium(II) chloride, acetato(2'-di-tert-butylphosphino-1,1'-biphenyl-2-yl)palladium(II), (1,2-Bis(diphenylphosphino)ethane)dichloropalladium(II), Bis[1,2-bis(diphenylphosphino)ethane]palladium (0), [(2S,3S)-Bis(diphenylphosphino)-butane] [eta3-allyl]palladium(II) perchlorate, and 1,3-bis(2,4,6-trimethylphenyl)-imidazol-2-ylidene(1,4-naphthoqui none)palladium (0) dimer.

6. The process according to claim 1, wherein the copper catalyst is selected from: copper (I) halogen salts and copper oxides.

7. The process according to claim 6, wherein the copper catalyst is selected from:. copper (I) chloride, copper (I) bromide, copper (I) iodide and copper (I) oxide.

8. The process according to claims 1 to 7, wherein the base is selected out of the group of carbonates, phosphates, fluorides, alkoxides and hydroxides with a suitable metal as counter ion.

9. The process according to claim 8, wherein the base is selected out of the group: potassium carbonate, potassium phosphate and caesium carbonate.

10. The process according to claims 1 to 9, wherein the aprotic solvent is selected out of the group: benzene, toluene, xylene, mesitylene, acetonitrile, tetrahydrofurane, dimethylformamide, n-methylpyrrolodinone, dimethylacetamide, dimethylsulfoxide, (2-methoxyethyl)ether and pyridine.

11. The process according to claims 1 to 10, wherein the reaction between the compound of formula II and formula III is carried out in the temperature range from 60 °C to 150 °C, preferably from 70 °C to 90 °C.

12. The process according to claims 1 to 11, wherein the second solvent is selected out of the group: methanol, ethanol, propanol, acetic acid, methylene chloride, dimethylformamide, tetrahydrofurane, pyridine, β-xylene, ethylacetate, benzene, toluene, xylene, mesitylene and acetonitrile.

13. The process according to claim 12, wherein the second solvent is selected out of the group: methanol, ethanol; acetic acid, methylene chloride, dimethylformamide, pyridine and *p*-xylene.

14. The process according to claims 1 to 13, wherein the reducing reagent is selected out of the group: H₂/Raney-Ni, H₂/Pd-C, H₂/PtO₂, H₂/Ru, NaBH₄/NiCl₂, NaBH₄/FeCl₂, HgPO₂/Pd-C, Sn/HCl, SnCl₂/HCl, Fe/HOAc, Fe/HCl, FeSO₄/HCl, Fe/FeSO₄, Zn/HCl, Na₂S, and Na₂S₂O₄.

## Patentansprüche

1. Verfahren zur Herstellung einer Verbindung der Formel I 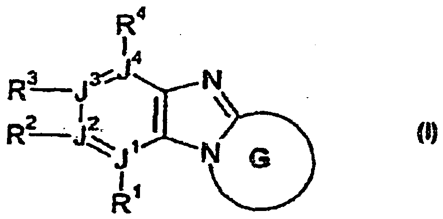 und/oder aller stereoisomerer Formen der Verbindung der Formel I und/oder von Gemischen dieser Formen in beliebigem Verhältnis und/oder eines physiologisch verträglichen Salzes der Verbindung der Formel I, worin
J1, J2, J3 und J4 unabhängig voneinander unter Kohlenstoff- oder Stickstoffatomen ausgewählt sind und zusammen mit den Kohlenstoffatomen, an die sie gebunden sind, einen stabilen aromatischen oder heteroaromatischen Ring bilden,
G unter Azetidin, Azepan, Azocan, Azabicyclo-[2.2.1]heptan, Azabicyclo[2.2.2]octan, Azacyclooctanon, Azacyclononanon, Azatricyclo-[4.3.1.1*3,8*]undecan, 4,4-Dimethyl-3,5-dioxaazatricyclo[5.2.1.0*2,6*]decan, 3,5-Dioxaazatricyclo-[5.2.1.0*2,6*]decan, 4,4-Dimethyl-3,5-dioxaazatricyclo[5.2.1.0*2,6*]decan-9-on, Azocan-2-on, Azonan, 1,4-Diazepan, [1,4]Diazocan, [1,2]Diazocan-3-on, [1,3]Diazocan-2-on, Imidazolin, Imidazolidin, Isothiazolidin, Isoxazolidin, Ketopiperazin, Morpholin, [1,4]Oxazocan, [1,3]Oxazocan-2-on, Piperazin, Piperidin, Pyrazolin, Pyrazolidin, 1,2-Dihydropyridin, Pyrrolidin, Pyrrolidinon, 2,3-Dihydro-1H-pyrrol, Pyrrolin, 5,6,7,8-Tetrahydro-1H-azocin-2-on, Tetrahydropyridin, Thiadiazin, Thiazolidin, Thiazolin oder Thiomorpholin ausgewählt ist,
wobei G unsubstituiert oder ein-, zwei-, drei- oder vierfach unabhängig voneinander durch Oxo oder R5 substituiert ist,
R1, R2, R3, R4 und R5 unabhängig voneinander gleich oder verschieden sind und für
a) ein Wasserstoffatom,
b) -(C₁-C₄)-Alkyl, wobei Alkyl unsubstituiert oder ein- bis dreifach durch R13 substituiert ist,
c) Halogen,
d) Phenyloxy-, wobei Phenyloxy unsubstituiert oder ein- bis dreifach durch R13 substituiert ist,
e) -(C₁-C₃-Fluoralkyl,
f) -N(R10)-(C₁-C₄)-Alkyl, wobei Alkyl unsubstituiert oder ein- bis dreifach durch R13 substituiert ist,
g) -(C₆-C₁₄) -Aryl, wobei Aryl unsubstituiert oder ein-, zwei-, drei- oder vierfach unabhängig voneinander durch R13 substituiert ist,
h) -(C₄-C₁₄)-Heteroaryl, wobei Heteroaryl unsubstituiert oder ein-, zwei-, drei- oder vierfach unabhängig voneinander durch R13 substituiert ist,
i) -(C₃-C₈)-Cycloalkyl, wobei Cycloalkyl unsubstituiert oder ein-, zwei-, drei- oder vierfach unabhängig voneinander durch R13 substituiert ist,
j) einen 3- bis 7-gliedrigen cyclischen Rest mit 1, 2, 3 oder 4 unter Stickstoff, Schwefel oder Sauerstoff ausgewählten Heteroatomen, wobei der cyclische Rest unsubstituiert oder ein-, zwei-, drei- oder vierfach unabhängig voneinander durch R13 substituiert ist,
k) -O-CF₃,
l) -O-(C₁-C₄)-Alkyl, wobei Alkyl unsubstituiert oder ein- bis dreifach durch R13 substituiert ist,
m) -NO₂ ,
n) -CN,
o) -OH,
p) -C(O)-R10,
q) -C(O)-O-R11,
r) -C(O)-N(R11)-R12,
s) -N(R11)-R12,
t) -N(R10)-SO2-R10,
v) -S-R10,
w) -SOₙ-R10, worin n für 1 oder 2 steht,
x) -SO₂-N(R11)-R12
stehen oder
y) in dem Fall, daß eine oder mehrere der Variablen J1, J2, J3 oder J4 für ein Stickstoffatom stehen, mindestens eine der Variablen R1, R2, R3 und R4 fehlt, oder
R1 und R2, R2 und R3 oder R3 und R4 zusammen mit den Atomen, an die sie gebunden sind, einen 5- bis 8-gliedrigen Ring mit 0, 1, 2, 3 oder 4 unter Stickstoff, Schwefel oder Sauerstoff ausgewählten Heteroatomen bilden, wobei der Ring unsubstituiert oder ein-, zwei-, drei- oder vierfach durch R14 substituiert ist,
R10 für ein Wasserstoffatom, -(C₁-C₃)-Fluoralkyl oder -(C₁-C₆)-Alkyl steht,
R11 und R12 unabhängig voneinander gleich oder verschieden sind und für
a) ein Wasserstoffatom,
b) -(C₁-C₆)-Alkyl, wobei Alkyl unsubstituiert oder ein-, zwei- oder dreifach unabhängig voneinander durch R13 substituiert ist, _{C}) -(C₆-C₁₄)-Aryl, wobei Aryl unsubstituiert oder ein-, zwei- oder dreifach unabhängig voneinander durch R13 substituiert ist, d) -(C₄-C₁₄)-Heteroaryl, wobei Heteroaryl unsubstituiert oder ein-, zwei- oder dreifach unabhängig voneinander durch R13 substituiert ist,
stehen,
R13 für Halogen, -NO₂, -CN, =O -OH, -(C₁-C₈)-Alkyl, -(C₁-C₈)-Alkoxy, -CF₃ , Phenyloxy-, -C(O)-R10, -C(O)-O-R17, -C(O)-N(R17)-R18, -N(R17)-R18, -N(R10)-SO₂-R10, -S-R10, -SOₙ-R10, worin n für 1 oder 2 steht, -SO₂-N(R17)-R18, -(C₆-C₁₄)-Aryl, wobei Aryl unsubstituiert oder ein-, zwei- oder dreifach unabhängig voneinander durch R14 substituiert ist, -(C₄-C₁₄)-Heteroaryl, wobei Heteroaryl unsubstituiert oder ein-, zwei- oder dreifach unabhängig voneinander durch R14 substituiert ist, -(C₃-C₈)-Cycloalkyl, wobei das Cycloalkyl unsubstituiert oder ein-, zwei- oder dreifach unabhängig voneinander durch R14 substituiert ist, oder einen 3- bis 7-gliedrigen cyclischen Rest mit 1, 2, 3 oder 4 unter Stickstoff, Schwefel oder Sauerstoff ausgewählten Heteroatomen, wobei der cyclische Rest unsubstituiert oder ein-, zwei- oder dreifach unabhängig voneinander durch R14 substituiert ist, steht,
R14 für Halogen, -OH, =O, -CN, -CF₃, -(C₁-C₈)-Alkyl, -(C₁-C₄)-Alkoxy, -NO₂ -C(O)-OH, -NH₂, -C(O)-O-(C₁-C₄)-Alkyl, -(C₁-C₈)-Alkylsulfonyl, -C(O)-NH-(C₁-C₈)-Alkyl, -C (O)-N[ (C₁-C₈)-Alkyl]₂, -C (O)-NH₂, -S-R10, -N(R10)-C(O)-NH-(C₁-C₈)-Alkyl oder -N(R10)-C(O)-N[(C₁-C₈)-Alkyl]₂ steht,
R17 und R18 unabhängig voneinander gleich oder verschieden sind und für
a) ein Wasserstoffatom,
b) -(C₁-C₆)-Alkyl,
c) -(C₆₋C₁₄)-Aryl oder
d) - (C₄-C₁₄) -Heteroaryl
stehen,
bei dem man eine Verbindung der Formel II 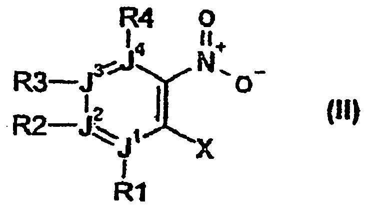 worin R1, R2, R3, R4, J1, J2, J3 und J4 die bei Formel I angegebene Bedeutung besitzen und
X für Cl, Br, I, Triflat oder Nonaflat steht, in Gegenwart eines unter Palladium und Kupfer ausgewählten Metallkatalysators, einer Base, eines Liganden, wobei der Ligand dann, wenn es sich bei dem Metallkatalysator um Palladium handelt, aus der Gruppe (+/-)-2,2'-Bis(diphenylphosphino)-1,1'-binaphthalin, (*R*)-(-)-1-[(*S*)-2-(Diphenylphosphino)ferrocenyl]ethyldicyclohexylphosphin, 1,2-Bis(diphenylphosphino)ethan, 1,3-Bis(diphenylphosphino)propan, (*R*)-(-)-1-[(*S*)-2-(Dicyclohexylphosphino)ferrocenyl]ethyldi-*tert.*-butylphosphin, (*R*)-(+)-1,1'-Bis(diphenylphosphino)-2,2'-bis(*N*,*N-*diisopropylamido)ferrocen, (*S*,*S*)-1-[1-(Di-tert.-butylphosphino)ethyl]-2-(diphenylphosphino)ferrocen, (1*R*,2*R*)-(+)-1,2-Diaminocyclohexan-*N*,*N*'-bis(2-diphenylphosphino)-1-naphthoyl, (-)-1,2-Bis-((2*S*,5*S*)-2,5-Diisopropylphospholano)benzol, Bis-[(2-diphenylphosphino)phenyl]ether, (*S*)-(-)-2,2'-Bis(di-para-tolylphosphino)-1,1'-binaphthyl, 4,5-Bis(bis(3,5-bis(trifluormethyl)phenyl)phosphino)-9,9-dimethylxanthen, 2,2'-Bis[(2',4',6'-triisopropyl)dicyclohexylphosphino]biphenyl, 2,2'-Bis-(di-*tert*.-butylphosphino)biphenyl und Tri-*tert*.-butylphosphin ausgewählt wird oder der Ligand dann, wenn es sich bei dem Metallkatalysator um Kupfer handelt, aus der Gruppe Ethylendiamin, *N-*Methylethylendiamin, *N*,*N*-Dimethylethan-1,2-diamin, N-Butylethylendiamin, *N*,*N*-Dimethylethylendiamin, *N*,*N*,*N*'-Trimethylendiamin, *N*,*N*,*N*,*N*'-Tetramethylethylendiamin, trans-1,2-Cyclohexandiamin, *cis-*1,2-Cyclohexandiamin, *cis*/*trans*-1,2-Cyclohexandiamin, *N*,*N*'-Diethyl-1,2-cyclohexandiamin, *N,N'-*Dipropyl-1,2-cyclohexandiamin, 1,3-Propylendiamin, 1,2-Benzoldiamin, Phenanthridin, Acridin, Acridinorange, 9-Aminoacridin, 9-Hydroxy-4-methoxyacridin, Proflavin, 4-(2-Pyriylazo)resorcinol, 1,2-Dihydro-1-(2-(2-pyridyl)ethyl)-3,6-pyridazindion, [1,10]Phenanthrolin, 5-Nitro-[1,10]-phenanthrolin, Bathophenanthrolin, Spiramycin, Bicinchoninsäure-Natriumsalz (bca), 1-(4-Pyridyl)-pyridiniumchlorid, 2-Pyridylessigsäure-hydrochlorid, 8-Mercaptochinolin-hydrochlorid, Dimethylaminoessigsäure, Picolinsäure, 3-Hydroxypicolinsäure, 3-Hydroxypicolinamid, Glykol, Pyridin, 2-Aminopyridin, 2-Hydroxypyridin, 3-Cyanopyridin, 4-Cyanopyridin, 2-Ethylpyridin, 2-Amino-6-methylpyridin, 2-(Aminomethylpyridin), 2-(Hydroxymethylpyridin), 2-Hydroxy-6-methylpyridin, 2-Dimethylaminopyridin, 4-Dimethylaminopyridin, 2-(2-Hydroxyethyl)pyridin, 4-tert.-Butylpyridin, 3-Acetoxypyridin, 2-Phenylpyridin, 4-Phenylpyridin, 4-Benzoylpyridin, 2-(2-Thienyl)pyridin, 2-Benzylpyridin, 2-Anilinopyridin, 3-Pyridinpropanol, 1-(2-Pyridyl)piperazin, Di-2-pyridylketon, 2-Pyridylessigsäureethylester, 2-(2-Diethylaminoethyl)pyridin, 4-(2-Diethylaminoethyl)pyridin, 2,6-Di-tert.-butylpyridin, (*S*,*S*)-2,6-Bis(4-isopropyl-2-oxazolin-2-yl)pyridin, 2,3-Pyridindicarbonsäure, 2,6-Pyridindicarbonsäure, 3,5-Pyridindicarbonsäure, 1,3-Di(4-pyridyl)propan, 2,3-Di-3-pyridyl-2,3-butandiol, 2,2'-Bipyridin, 2,2-Dipyridyl, 4,4'-Dimethyl-2,2'-dipyridyl, 3-Hydroxypyridin, 2-Mercaptopyridin, 2-(2-Methylaminoethyl)pyridin, 3-Hydroxypicolinamin, 3-Hydroxypicolinsäure, 2,2':6',2"-Terpyridin, 2-Picolin, 6,6'-Bi-2-picolin, 2,4-Lutidin, 2,6-Lutidin-α-2,3-diol, 2,6-Lutidin, 2,4,6-Collidin, Picolinamid, Picolinsäureethylester, Isonicotinsäureethylester, Chinolin, 2-Phenylchinolin, 8-Hydroxychinolin, 8-Acetoxychinolin, 2-Methyl-8-nitrochinolin, 7,8-Benzochinolin, 2-Chinolinol, 2-Chinolinthiol, Chinolin-4-carbonsäure, 2-Phenyl-4-chinolincarbonsäure, 2,4-Hydroxychinolin-Mononatriumsalz, 8-Ethoxychinolin-5-sulfonsäure-Natriumsalz, 8-Hydroxy-5-nitrochinolin, 4-Chlor-7-(trifluormethyl)chinolin, 8-Hydroxychinolin-5-sulfonsäuremonohydrat, 5-Nitrochinaldinsäure, Isochinolin, Isochinolin-3-carbonsäure-hydrat, 1,4,5-Triazanaphthalin, Chinaldin, 4-Chlorchinaldin, Nicotin, Isonicotinamin, Neocuproin, Lysin, Cystin, α-Alanin, Arginin, Cystein und β-Alanin ausgewählt wird, und eines aprotischen Lösungsmittels mit einer Verbindung der Formel III 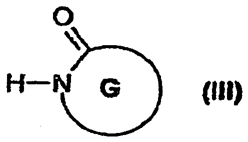 worin der Ring G die bei Formel I angegebene Bedeutung besitzt,
zu einer Verbindung der Formel IV 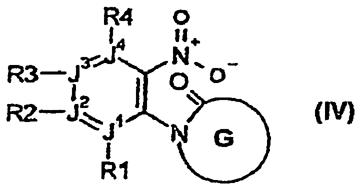 umsetzt und die Verbindung der Formel IV in Gegenwart eines Reduktionsmittels und eines zweiten Lösungsmittel in eine Verbindung der Formel I umwandelt und
gegebenenfalls die Verbindung der Formel I in ihr physiologisch verträgliches Salz umwandelt.

2. Verfahren nach Anspruch 1, bei dem man eine Verbindung der Formel I herstellt, worin J1, J2, J3 und J4 zusammen mit den Kohlenstoffatomen, an die sie gebunden sind, einen unter Benzol, Pyrazin, Pyridazin, Pyridin, Pyrimidin, Triazin oder Tetrazin ausgewählten Ring bilden,
G unter Azetidin, Azepan, Azocan, Azabicyclo-[2.2.1]heptan, Azabicyclo[2.2.2]octan, Azacyclooctanon, Azacyclononanon, Azatricyclo-[4.3.1.1*3,8*]undecan, 4,4-Dimethyl-3,5-dioxaazatricyclo[5.2.1.0*2,6*]decan, 3,5-Dioxaazatricyclo-[5.2.1.0*2,6*]decan, 4,4-Dimethyl-3,5-dioxaazatricyclo[5.2.1.0*2,6*]decan-9-on, Azocan-2-on, Azonan, 1,4-Diazepan, [1,4]Diazocan, [1,2]-Diazocan-3-on, [1,3]Diazocan-2-on, Imidazolin, Imidazolidin, Isothiazolidin, Isoxazolidin, Ketopiperazin, Morpholin, [1,4]Oxazocan, [1,3]-Oxazocan-2-on, Piperazin, Piperidin, Pyrazolin, Pyrazolidin, 1,2-Dihydropyridin, Pyrrolidin, Pyrrolidinon, 2,3-Dihydro-1H-pyrrol, Pyrrolin, 5,6,7,8-Tetrahydro-1H-azocin-2-on, Tetrahydropyridin, Thiadiazin, Thiazolidin, Thiazolin oder Thiomorpholin ausgewählt ist,
wobei G unsubstituiert oder ein-, zwei-, drei- oder vierfach unabhängig voneinander durch Oxo oder R5 substituiert ist,
R1, R2, R3, R4 und R5 unabhängig voneinander gleich oder verschieden sind und für
a) ein Wasserstoffatom,
b) F,
c) Cl,
d) -(C₁-C₄)-Alkyl, wobei Alkyl unsubstituiert oder ein- bis dreifach durch R13 substituiert ist,
e) - (C₁-C₃)-Fluoralkyl,
f) Phenyl, wobei Phenyl unsubstituiert oder einbis dreifach unabhängig voneinander durch R13 substituiert ist,
g) - (C₄-C₁₄) -Heteroaryl, wobei Heteroaryl unter Acridinyl, Azaindol (1H-Pyrrolopyridinyl), Azabenzimidazolyl, Azaspirodecanyl, Azepinyl, Azetidinyl, Benzimidazolyl, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Carbazolyl, 4aH-Carbazolyl, Carbolinyl, Chromanyl, Chromenyl, Cinnolinyl, Decahydrochinolinyl, 4,5-Dihydrooxazolinyl, Dioxazolyl, Dioxazinyl, 1,3-Dioxolanyl, 1,3-Dioxolenyl, 3,3-Dioxo[1,3,4]oxathiazinyl, 6H-1,5,2-Dithiazinyl, Dihydrofuro[2,3-b]tetrahydrofuranyl, Furanyl, Furazanyl, Imidazolidinyl, Imidazolinyl, Imidazolyl, Indanyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl, Isothiazolyl, Isothiazolidinyl, Isothiazolinyl, Isoxazolyl, Isoxazolinyl, Isoxazolidinyl, 2-Isoxazolinyl, Ketopiperazinyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, 1,2-Oxathiepanyl, 1,2-Oxathiolanyl, 1,4-Oxazepanyl, 1,4-Oxazepinyl, 1,2-Oxazinyl, 1,3-Oxazinyl, 1,4-Oxazinyl, Oxazolidinyl, Oxazolinyl, Oxazolyl, Oxetanyl, Oxocanyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyrazolidinyl, Pyrazolinyl, Pyrazolyl, Pyrazolo[3,4-b]pyridin, Pyridazinyl, Pyridooxazolyl, Pyridoimidazolyl, Pyridothiazolyl, Pyridinyl, Pyridyl, Pyrimidinyl, Pyrrolidinyl, Pyrrolidinonyl, Pyrrolinyl, 2H-Pyrrolyl, Pyrrolyl, Chinazolinyl, Chinolinyl, 4H-Chinolizinyl, Chinoxalinyl, Chinuclidinyl, Tetrahydrofuranyl, Tetrahydroisochinolinyl, Tetrahydrochinolinyl, Tetrahydrofuranyl, Tetrahydropyranyl, Tetrahydropyridinyl, Tetrahydrothiophenyl, Tetrazinyl, Tetrazolyl, 6H-1,2,5-Thiadiazinyl, 1,2,3-Thiadiazolyl, 1,2,4-Thiadiazolyl, 1,2,5-Thiadiazolyl, 1,3,4-Thiadiazolyl, Thianthrenyl, 1,2-Thiazinyl, 1,3-Thiazinyl, 1,4-Thiazinyl, 1,3-Thiazolyl, Thiazolyl, Thiazolidinyl, Thiazolinyl, Thienyl, Thietanyl, Thienothiazolyl, Thienooxazolyl, Thienoimidazolyl, Thietanyl, Thiomorpholinyl, Thiophenolyl, Thiophenyl, Thiopyranyl, 1,2,3-Triazinyl, 1,2,4-Triazinyl, 1,3,5-Triazinyl, 1,2,3-Triazolyl, 1,2,4-Triazolyl, 1,2,5-Triazolyl, 1,3,4-Triazolyl und Xanthenyl ausgewählt ist und unsubstituiert oder ein-, zwei-, drei- oder vierfach unabhängig voneinander durch R13 substituiert ist,
h) -(C₃-C₈)-Cycloalkyl, wobei Cycloalkyl unsubstituiert oder ein-, zwei-, drei- oder vierfach unabhängig voneinander durch R13 substituiert ist, oder
i) einen 3- bis 7-gliedrigen cyclischen Rest, der unter Azepin, Azetidin, Aziridin, Azirin, 1,4-Diazepan, 1,2-Diazepin, 1,3-Diazepin, 1,4-Diazepin, Diaziridin, Diazirin, Dioxazol, Dioxazin, Dioxol, 1,3-Dioxolen, 1,3-Dioxolan, Furan, Imidazol, Imidazolin, Imidazolidin, Isothiazol, Isothiazolidin, Isothiazolin, Isoxazol, Isoxazolin, Isoxazolidin, 2-Isoxazolin, Ketomorpholin, Ketopiperazin, Morpholin, 1,2-Oxathiepan, 1,2-Oxathiolan, 1,4-Oxazepan, 1,2-Oxazin, 1,3-Oxazin, 1,4-Oxazin, Oxazol, Oxaziridin, Oxetan, Oxiran, Piperazin, Piperidin, Pyran, Pyrazin, Pyrazol, Pyrazolin, Pyrazolidin, Pyridazin, Pyridin, Pyrimidin, Pyrrol, Pyrrolidin, Pyrrolidinon, Pyrrolin, Tetrahydrofuran, Tetrahydropyran, Tetrahydropyridin, Tetrazin, Tetrazol, Thiadiazin, Thiadiazol, 1,2-Thiazin, 1,3-Thiazin, 1,4-Thiazin, 1,3-Thiazol, Thiazol, Thiazolidin, Thiazolin, Thienyl, Thietan, Thiomorpholin, Thiopyran, 1,2,3-Triazin, 1,2,4-Triazin, 1,3,5-Triazin, 1,2,3-Triazol oder 1,2,4-Triazol ausgewählt ist und unsubstituiert oder ein-, zwei-, drei- oder vierfach unabhängig voneinander durch R13 substituiert ist,
j) -O-CF₃ ,
k) -O-(C₁-C₄)-Alkyl, wobei Alkyl unsubstituiert oder ein- bis dreifach durch R13 substituiert ist,
l) -N(R10)-(C₁-C₄)-Alkyl, wobei Alkyl unsubstituiert oder ein- bis dreifach durch R13 substituiert ist,
m) -CN,
n) -OH,
o) Phenyloxy-, wobei Phenyloxy unsubstituiert oder ein- bis dreifach durch R13 substituiert ist,
p) -C(O)-O-R11,
q) -C(O)-N(R11)-R12,
r) -N(R11)-R12,
s) -N(R10)-SO₂-R10,
t) -S-R10,
v) -SOₙ-R10, worin n für 1 oder 2 steht,
w) -SO₂-N(R11)-R12,
x) -C(O)-R10
stehen oder
y) in dem Fall, daß eine oder mehrere der Variablen J1, J2, J3 oder J4 für ein Stickstoffatom stehen, mindestens eine der Variablen R1, R2, R3 und R4 fehlt,
R10 für ein Wasserstoffatom, -(C₁-C₃)-Fluoralkyl oder -(C₁-C₆)-Alkyl steht,
R11 und R12 unabhängig voneinander gleich oder verschieden sind und für
a) ein Wasserstoffatom,
b) -(C₁-C₄)-Alkyl, wobei Alkyl unsubstituiert oder ein-, zwei- oder dreifach unabhängig voneinander durch R13 substituiert ist,
c) Phenyl, wobei Phenyl unsubstituiert oder ein-, zwei- oder dreifach unabhängig voneinander durch R13 substituiert ist,
d) -(C₄-C₁₄)-Heteroaryl, wobei Heteroaryl die oben angegebene Bedeutung besitzt und unsubstituiert oder ein-, zwei- oder dreifach unabhängig voneinander durch R13 substituiert ist,
stehen,
R13 für F, Cl, -CN, =O, -OH, -(C₁-C₈)-Alkyl, -(C₁-C₈)-Alkoxy, -CF₃ , Phenyloxy-, -C(O)-R10, -C(O)-O-R17, -C(O)-N(R17)-R18, -N(R17)-R18, -N(R10)-SO₂-R10, -S-R10, -SOₙ-R10, worin n für 1 oder 2 steht, -SO₂-N(R17)-R18, Phenyl, wobei Phenyl unsubstituiert oder ein-, zwei- oder dreifach unabhängig voneinander durch R14 substituiert ist, -(C₄-C₁₄)-Heteroaryl, wobei Heteroaryl die oben angegebene Bedeutung besitzt und unsubstituiert oder ein-, zwei- oder dreifach unabhängig voneinander durch R14 substituiert ist, -(C₃-C₆)-Cycloalkyl, wobei das Cycloalkyl unsubstituiert oder ein-, zwei- oder dreifach unabhängig voneinander durch R14 substituiert ist, oder einen 3- bis 7-gliedrigen cyclischen Rest, der die oben angegebene Bedeutung besitzt und unsubstituiert oder ein-, zwei- oder dreifach unabhängig voneinander durch R14 substituiert ist, steht,
R14 für F, Cl, -OH, =O, -CN, -CF₃, -(C₁-C₈₎-Alkyl, - (C₁-C₄) -Alkoxy, -C(O)-OH, -NH₂, -C (O)-O- (C₁-C₄)-Alkyl, - (C₁-C₈) -Alkylsulfonyl, -C(O)-NH₂, -C(O)-NH-(C₁-C₈) -Alkyl, -C(O) -N [(C₁-C₈)-Alkyl]₂, -S-R10, -N(R10)-C(O)-NH-(C₁-C₈)-Alkyl oder -N(R10)-C(O)-N[(C₁-C₈)-Alkyl]₂ steht,
R17 und R18 unabhängig voneinander gleich oder verschieden sind und für
a) ein Wasserstoffatom,
b) - (C₁-C₄) -Alkyl,
c) Phenyl oder
d) -(C₄-C₁₄)-Heteroaryl, wobei Heteroaryl die oben angegebene Bedeutung besitzt,
stehen und
X für Cl, Br oder I steht.

3. Verfahren nach Anspruch 1, bei dem man eine der folgenden Verbindungen der Formel I herstellt:
2,3-Dihydro-1*H*-benzo[d]pyrrolo[1,2-a]imidazol;
7-Methyl-2,3-dihydro-1*H*-benzo[d]pyrrolo[1,2-a]-imidazol;
6-Methyl-2,3-dihydro-1*H*-benzo[d]pyrrolo[1,2-a]-imidazol;
7-Methoxy-2,3-dihydro-1*H*-benzo[d]pyrrolo[1,2-a]-imidazol;
5-Methyl-2,3-dihydro-1*H*-benzo[d]pyrrolo[1,2-a]-imidazol-6-carbonsäuremethylester;
2-Methoxy-7,8-dihydro-6*H*-pyrrolo[2',1':2,3]-imidazo[4,5-b]pyridin;
2,6-Dimethyl-7,8-dihydro-6*H*-pyrrolo[2',1':2,3]-imidazo[4,5-b]pyridin; 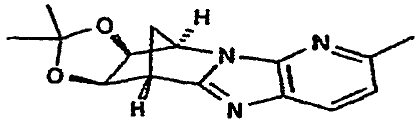
1,2,3,4-Tetrahydrobenzo[4,5]imidazo[1,2-a]pyridin;
3,9-Dimethyl-6,7,8,9-tetrahydrodipyrido[1,2-a:3',2'-d]imidazol;
7-Chlor-4,4-diphenyl-1,2,3,4-tetrahydrobenzo[4,5]-imidazo[1,2-a]pyridin;
Dimethyl-(*S*)-7,8,9,10-tetrahydro-6*H*-benzo[4,5]-imidazo[1,2-a]azepin-6-ylamin;
3-Methyl-5,6,7,8,9,10-hexahydro-4,4b,11-triazacycloocta[a]inden;
2-Methyl-6,7,8,9,10,11-hexahydro-5*H*-4,4b,12-triazacyclonona[a]inden;
3-Methyl-8,8-diphenyl-6,7,8,9-tetrahydrodipyrido-[1,2-a:3',2'-d]imidazol; 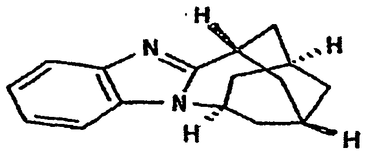
2-Methyl-8,8-diphenyl-6,7,8,9-tetrahydrodipyrido-[1,2-a:3',2'-d]imidazol;
5,6,7,8,9,10-Hexahydro-1,4b,11-triazacycloocta[a]-inden oder
3-Methoxy-6,7,8,9,10,11-hexahydro-5*H*-4b,12-diazacyclonona[a]inden.

4. Verfahren nach Anspruch 1, bei dem man den Palladiumkatalysator unter Pd-Alkanoaten, Pd-Alkanoatkomplexen, Pd-Acetonaten, Pd-Halogeniden, Pd-Halogenidkomplexen und Pd-Phosphinkomplexen auswählt.

5. Verfahren nach Anspruch 4, bei dem man den Palladiumkatalysator aus der Gruppe Palladium(II)-acetat, Palladium(II)-trifluoracetat, Tris-(dibenzylidenaceton)dipalladium(0), Tris-(dibenzylidenaceton)dipalladium(0)-Chloroformaddukt, Palladium(II)-chlorid, 2,2'-Bis(diphenylphosphino)-1,1'-binaphthyl-palladium(II)-chlorid, Acetato(2'-di-tert.-butylphosphino-1,1'-biphenyl-2-yl)palladium(II), (1,2-Bis(diphenylphosphino)-ethan)dichloropalladium(II), Bis[1,2-bis(diphenylphosphino)ethan]palladium(0), [(2*S*,3*S*)-Bis(diphenylphosphino)butan-[eta3-allyl]palladium-(II)-perchlorat und 1,3-Bis(2,4,6-trimethylphenyl)imidazol-2-yliden(1,4-naphthochinon)palladium(0)-Dimer auswählt.

6. Verfahren nach Anspruch 1, bei dem man den Kupferkatalysator unter Kupfer(I)-halogensalzen und Kupferoxiden auswählt.

7. Verfahren nach Anspruch 6, bei dem man den Kupferkatalysator unter Kupfer(I)-chlorid, Kupfer(I)-bromid, Kupfer(I)-iodid und Kupfer(I)-oxid auswählt.

8. Verfahren nach den Ansprüchen 1 bis 7, bei dem man die Base aus der Gruppe der Carbonate, Phosphate, Fluoride, Alkoxide und Hydroxide mit einem geeigneten Metall als Gegenion auswählt.

9. Verfahren nach Anspruch 8, bei dem man die Base aus der Gruppe Kaliumcarbonat, Kaliumphosphat und Caesiumcarbonat auswählt.

10. Verfahren nach den Ansprüchen 1 bis 9, bei dem man das aprotische Lösungsmittel aus der Gruppe Benzol, Toluol, Xylol, Mesitylen, Acetonitril, Tetrahydrofuran, Dimethylformamid, N-Methylpyrrolidinon, Dimethylacetamid, Dimethylsulfoxid, (2-Methoxyethyl)ether und Pyridin auswählt.

11. Verfahren nach den Ansprüchen 1 bis 10, bei dem man die Umsetzung zwischen der Verbindung der Formel II und der Verbindung der Formel III im Temperaturbereich von 60°C bis 150°C, vorzugsweise 70°C bis 90°C, durchführt.

12. Verfahren nach den Ansprüchen 1 bis 11, bei dem man das zweite Lösungsmittel aus der Gruppe Methanol, Ethanol, Propanol, Essigsäure, Methylenchlorid, Dimethylformamid, Tetrahydrofuran, Pyridin, *p*-Xylol, Essigsäureethylester, Benzol, Toluol, Xylol, Mesitylen und Acetonitril auswählt.

13. Verfahren nach Anspruch 12, bei dem man das zweite Lösungsmittel aus der Gruppe Methanol, Ethanol, Essigsäure, Methylenchlorid, Dimethylformamid, Pyridin und *p*-Xylol auswählt.

14. Verfahren nach den Ansprüchen 1 bis 13, bei dem man das Reduktionsmittel aus der Gruppe H₂/Raney-Ni, H₂/Pd-C, H₂/PtO₂, H₂/Ru, NaBH₄/NiCl₂, NaBH₄/FeCl₂, H₃PO₂/Pd-C, Sn/HCl, SnCl₂/HCl, Fe/HOAc, Fe/HCl, FeSO₄/HCl, Fe/FeSO₄, Zn/HCl, Na₂S und Na₂S₂O₄ auswählt.

## Revendications

1. Procédé pour préparer un composé de formule I 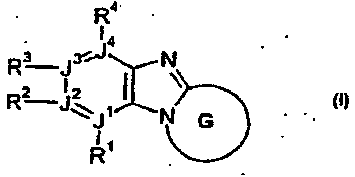 et/ou toutes les formes stéréoisomères du composé de formule I, et/ou les mélanges de ces formes dans toutes proportions, et/ou un sel physiologiquement toléré du composé de formule I, dans lequel
J1, J2, J3 et J4, indépendamment les uns des autres, sont choisis parmi les atomes de carbone et d'azote et forment, conjointement avec les atomes de carbone auxquels ils sont attachés, un noyau aromatique ou hétéroaromatique stable,
G est choisi parmi l'azétidine, l'azépane, l'azocane, l'aza-bicyclo[2.2.1]heptane, l'aza-bicyclo[2.2.2]octane, l'azacyclooctanone, l'azacyclononanone, l'aza-tricyclo [4.3.1.1*3,8*]undécane, le 4,4-diméthyl-3,5-dioxaazatricyclo[5.2.1.0*2,6*]-décane, le 3,5-dioxaazatricyclo[5.2.1.0*2,6*]décane, la 4,4-diméthyl-3,5-dioxa-azatricyclo[5.2.1.0*2,6*]décan-9-one, l'azocane-2-one, l'azonane, le 1,4-diazépane, le [1,4]diazocane, la [1,2]diazocan-3-one, la [1,3]diazocan-2-one, l'imidazoline, l'imidazolidine, l'isothiazolidine, l'isoxazolidine, la cétopipérazine, la morpholine, le [1,4]oxazocane, la [1,3]oxazocan-2-one, la pipérazine, la pipéridine, la pyrazoline, la pyrazolidine, la 1,2-dihydro-pyridine, la pyrrolidine, la pyrrolidinone, le 2,3-dihydro-1*H*-pyrrole, la pyrroline, la 5,6,7,8-tétrahydro-1*H*-azocin-2-one, la tétrahydropyridine, la thiadiazine, la thiazolidine, la thiazoline et la thiomorpholine, G étant non substitué ou substitué une, deux, trois ou quatre fois indépendantes les unes des autres par un groupe oxo ou par R5,
R1, R2, R3, R4 et R5, indépendamment les uns des autres, sont identiques ou différents et représentent
a) un atome d'hydrogène,
b) un groupe alkyle en C₁-C₄, le groupe alkyle étant non substitué ou substitué une à trois fois par R13,
c) un halogène,
d) un groupe phényloxy, le groupe phényloxy étant non substitué ou substitué une à trois fois par R13,
e) un groupe fluoroalkyle en C₁-C₃,
f) un groupe -N(R10)-alkyle en C₁-C₄, le groupe alkyle étant non substitué ou substitué une à trois fois par R13,
g) un groupe aryle en C₆-C₁₄, le groupe aryle étant non substitué ou substitué une, deux, trois ou quatre fois indépendantes les unes des autres par R13,
h) un groupe hétéroaryle en C₄-C₁₄, le groupe hétéroaryle étant non substitué ou substitué une, deux, trois ou quatre fois indépendantes les unes des autres par R13,
i) un groupe cycloalkyle en C₃-C₈, ledit groupe cycloalkyle étant non substitué ou substitué une, deux, trois ou quatre fois indépendantes les unes des autres par R13,
j) un résidu cyclique à 3 à 7 chaînons, contenant 1, 2, 3 ou 4 hétéroatomes choisis parmi l'azote, le soufre et l'oxygène, ledit résidu cyclique étant non substitué ou substitué une, deux, trois ou quatre fois indépendantes les unes des autres par R13,
k) -O-CF₃ ,
l) un groupe -O-alkyle en C₁-C₄, le groupe alkyle étant non substitué ou substitué une à trois fois par R13,
m) -NO₂,
n) -CN,
o) -OH,
p) -C(O)-R10,
q) -C(O)-O-R11,
r) -C(O)-N(R11)-R12,
s) -N(R11)-R12,
t) -N(R10)-SO₂-R10,
v) -S-R10,
w) -SOₙ-R10, n valant 1 ou 2,
x) -SO₂-N(R11)-R12 , ou
y) l'un au moins de R1, R2, R3 et R4 est absent dans le cas où l'un ou plusieurs de J1, J2, J3 et J4 représentent un atome d'azote, ou bien
R1 et R2, R2 et R3 ou R3 et R4 forment conjointement avec les atomes auxquels ils sont attachés un cycle à 5 ou 8 chaînons, contenant jusqu'à 0, 1, 2, 3 ou 4 hétéroatomes choisis parmi l'azote, le soufre et l'oxygène, ledit cycle étant non substitué ou substitué une, deux, trois ou quatre fois par R14,
R10 représente un atome d'hydrogène, un groupe fluoroalkyle en C₁-C₃ ou un groupe alkyle en C₁-C₆,
R11 et R12, indépendamment l'un de l'autre, sont identiques ou différents et représentent
a) un atome d'hydrogène,
b) un groupe alkyle en C₁-C₆, le groupe alkyle étant non substitué ou substitué une, deux ou trois fois indépendantes les unes des autres par R13,
c) un groupe aryle en C₆-C₁₄, le groupe aryle étant non substitué ou substitué une, deux ou trois fois indépendantes les unes des autres par R13, ou
d) un groupe hétéroaryle en C₄-C₁₄, le groupe hétéroaryle étant non substitué ou substitué une, deux ou trois fois indépendantes les unes des autres par R13, R13 représente un halogène, un groupe -NO₂, -CN, =O,-OH, alkyle en C₁-C₈, alcoxy en C₁-C₈, -CF₃, phényloxy,-C(O)-R10, -C(O)-O-R17, -C(O)-N(R17)-R18, -N(R17)-R18,-N(R10)-SO₂-R10, -S-R10 , -SOₙ-R10 , n valant 1 ou 2, -SO₂-N(R17)-R18, aryle en C₆-C₁₄, le groupe aryle étant non substitué ou substitué une, deux ou trois fois indépendantes les unes des autres par R14, hétéroaryle en C₄-C₁₄, le groupe hétéroaryle étant non substitué ou substitué une, deux ou trois fois indépendantes les unes des autres par R14, cycloalkyle en C₃-C₈, ledit groupe cycloalkyle étant non substitué ou substitué une, deux ou trois fois indépendantes les unes des autres par R14, ou un résidu cyclique à 3 à 7 chaînons, contenant 1, 2, 3 ou 4 hétéroatomes choisis parmi l'azote, le soufre et l'oxygène, ledit résidu cyclique étant non substitué ou substitué une, deux ou trois fois indépendantes les unes des autres par R14,
R14 représente un halogène, un groupe -OH, =O, -CN, -CF₃, alkyle en C₁-C₈, alcoxy en C₁-C₄, -NO₂, -C(O)-OH, -NH₂, -C(O)-O-alkyle en C₁-C₄, (alkyl en C₁-C₈) sulfonyle, -C(O)-NH-alkyle en C₁-C₈, -C(O)-N-[alkyle en C₁-C₈]₂, -C(O)-NH₂, -S-R10, -N(R10)-C(O)-NH-alkyle en C₁-C₈, ou -N(R10)-C(O)-N-[alkyle en C₁-C₈]₂,
R17 et R18, indépendamment l'un de l'autre, sont identiques ou différents et représentent
a) un atome d'hydrogène,
b) un groupe alkyle en C₁-C₆,
c) un groupe aryle en C₆-C₁₄, ou
d) un groupe hétéroaryle en C₄-C₁₄,
ledit procédé comprenant une réaction d'un composé de formule II 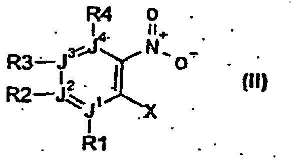
dans laquelle R1, R2, R3, R4, J1, J2, J3 et J4 sont tels que définis dans la formule I, et
X représente Cl, Br, I, un triflate ou un nonaflate, avec un composé de formule III 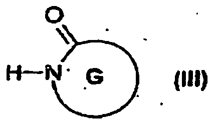 dans laquelle le cycle G est tel que défini dans la formule I,
en présence d'un catalyseur métallique choisi parmi le palladium et le cuivre, d'une base, d'un ligand,
le ligand étant choisi dans le groupe suivant : (+/-)-2,2'-bis(diphénylphosphino)-1,1'-binaphtalène, (*R*)-(-)-1-[(*S*)-2-(diphénylphosphino)ferrocényl]éthyldicyclohexylphosphine, 1,2-bis(diphénylphosphino)éthane, 1,3-bis(diphénylphosphino)propane, (*R*)-(-)-1-[(*S*)-2-(dicyclohexylphosphino)-ferrocényl]éthyldi-*tert-*butylphosphine, (*R*)-(+)-1,1'-bis(diphénylphosphino)-2,2'-bis(*N*,*N*-diisopropylamido)ferrocène, (*S*,*S*)-1-[1-(di-*tert*-butylphosphino)éthyl]-2-(diphénylphosphino)-ferrocène, (1*R*,2*R*)-(+)-1,2-diaminocyclohexane-N,N'-bis(2-diphénylphosphino-1-naphtoyle, (-)-1,2-bis((2*S*,5*S*)-2,5-diisopropylphospholano)-benzène, bis[(2-diphénylphosphino)phényl]éther, (*S*)-(-)-2,2'-bis(di-para-tolylphosphino)-1,1'-binaphtyle, 4,5-bis(bis(3,5-bis(trifluorométhyl)phényl)-phosphino)-9,9-diméthylxanthène, 2,2'-bis[(2',4',6'-triisopropyl)-dicyclohexyl-phosphino]biphényle, 2,2'-bis(di-*tert-*butylphosphino)biphényle, et tri-*tert*-butylphosphine, si le catalyseur métallique est le palladium, ou le ligand étant choisi dans le groupe suivant :
éthylènediamine, *N*-méthyléthylènediamine, *N,N*-diméthyléthane-1,2-diamine, *N*-butyléthylènediamine, *N,N-*diméthyléthylènediamine, *N*,*N*,*N*'-triméthyléthylènediamine, *N,N,N,N*'-tétraméthyléthylènediamine, *trans*1,2-cyclohexanodiamine, *cis*-1,2-cyclohexanodiamine, *cis*/*trans*-1,2-cyclohexanodiamine, *N,N*'-diéthyl-1,2-cyclohexanodiamine, *N*,*N'*-dipropyl-1,2-cyclohexanodiamine, 1,3-propylènediamine, 1,2-benzènediamine, phénanthridine, acridine, orange d'acridine, 9-aminoacridine, 9-hydroxy-4-méthoxyacridine, proflavine, 4-(2-pyridylazo)résorcinol, 1,2-dihydro-1-(2-(2-pyridyl)-éthyl)-3,6-pyridazinedione, [1,10]phénanthroline, 5-nitro-[1,10]phénanthroline, bathophénanthroline, spiramycine, sel de sodium d'acide bicinchonique (bca), chlorure de 1-(4-pyridyl)pyridinium, chlorhydrate d'acide 2-pyridylacétique, chlorhydrate de 8-mercaptoquinoline, acide diméthylaminoacétique, acide picolinique, acide 3-hydroxypicolinique, 3-hydroxypicolinamide, glycol, pyridine, 2-aminopyridine, 2-hydroxypyridine, 3-cyanopyridine, 4-cyanopyridine, 2-éthylpyridine, 2-amino-6-méthylpyridine, 2-(aminométhylpyridine), 2-(hydroxyméthylpyridine), 2-hydroxy-6-méthylpyridine, 2-diméthylaminopyridine, 4-diméthylaminopyridine, 2-(2-hydroxyéthyl)pyridine, 4-tert-butylpyridine, 3-acétoxypyridine, 2-phénylpyridine, 4-phénylpyridine, 4-benzoylpyridine, 2-(2-thiényl)-pyridine, 2-benzylpyridine, 2-anilinopyridine, 3-pyridinepropanol, 1-(2-pyridyl)pipérazine, di-2-pyridylcétone, 2-pyridylacétate d'éthyle, 2-(2-diéthylaminoéthyl)-pyridine, 4-(2-diéthylaminoéthyl)-pyridine, 2,6-di-*tert*-butylpyridine, (*S*,*S*)-2,6-bis(4-isopropyl-2-oxazolin-2-yl)pyridine, acide 2,3-pyridine-dicarboxylique, acide 2,6-pyridine-dicarboxylique, acide 3,5-pyridine-dicarboxylique, 1,3-di(4-pyridyl)-propane, 2,3-di-3-pyridyl-2,3-butanediol, 2,2'-bipyridine, 2,2-dipyridyle, 4,4'-diméthyl-2,2'-dipyridyle, 3-hydroxypyridine, 2-mercaptopyridine, 2-(2-méthylaminoéthyl)pyridine, 3-hydroxypicolinamine, acide 3-hydroxypicolinique, 2,2':6',2"-terpyridine, 2-picoline, 6,6'-bi-2-picoline, 2,4-lutidine, 2,6-lutidine-α-2,3-diol, 2,6-lutidine 2,4,6-collidine, picolinamide, picolinate d'éthyle, isonicotinate d'éthyle, quinoline, 2-phénylquinoline, 8-hydroxyquinoline, 8-acétoxyquinoline, 2-méthyl-8-nitroquinoline, 7,8-benzoquinoline, 2-quinolinol, 2-quinolinethiol, acide quinoline-4-carboxylique, acide 2-phényl-4-quinoline-carboxylique, sel monosodique de 2,4-hydroxyquinoline, sel de sodium d'acide 8-éthoxyquinoline-5-sulfonique, 8-hydroxy-5-nitroquinoline, 4-chloro-7-(trifluorométhyl)quinoline, monohydrate d'acide 8-hydroxyquinoline-5-sulfonique, acide 5-nitroquinaldique, isoquinoline, hydrate d'acide isoquinoline-3-carboxylique, 1,4,5-triazanaphtalène, quinaldine, 4-chloroquinaldine, nicotine, isonicotinamine, néocuproïne, lysine, cystine, α-alanine, arginine, cystéine, et β-alanine, si le catalyseur métallique est le cuivre,
et d'un solvant aprotique, pour obtenir un composé de formule IV 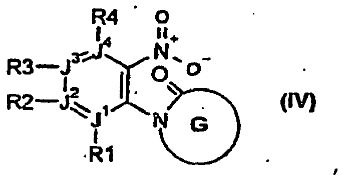
et la transformation du composé de formule IV en un composé de formule I en présence d'un réactif réducteur et d'un deuxième solvant,
le composé de formule I étant éventuellement transformé en son sel physiologiquement toléré.

2. Procédé selon la revendication 1, dans lequel un composé de formule I est préparé, dans lequel J1, J2, J3 et J4 forment conjointement avec les atomes de carbone auxquels ils sont attachés un cycle choisi parmi le benzène, la pyrazine, la pyridazine, la pyridine, la pyrimidine, la triazine et la tétrazine,
G est choisi parmi l'azétidine, l'azépane, l'azocane, l'aza-bicyclo[2.2.1]heptane, l'aza-bicyclo[2.2.2]octane, l'azacyclooctanone, l'azacyclononanone, l'aza-tricyclo [4.3.1.1*3,8*]undécane, le 4,4-diméthyl-3,5-dioxaazatricyclo[5.2.1.0*2,6*]-décane, le 3,5-dioxaazatricyclo[5.2.1.0*2,6*]décane, la 4,4-diméthyl-3,5-dioxa-azatricyclo[5.2.1.0*2,6*]décan-9-one, l'azocane-2-one, l'azonane, le 1,4-diazépane, le [1,4]diazocane, la [1,2]diazocan-3-one, la [1,3]diazocan-2-one, l'imidazoline, l'imidazolidine, l'isothiazolidine, l'isoxazolidine, la cétopipérazine, la morpholine, le [1,4]oxazocane, la [1,3]oxazocan-2-one, la pipérazine, la pipéridine, la pyrazoline, la pyrazolidine, la 1,2-dihydro-pyridine, la pyrrolidine, la pyrrolidinone, le 2,3-dihydro-1*H*-pyrrole, la pyrroline, la 5,6,7,8-tétrahydro-1*H*-azocin-2-one, la tétrahydropyridine, la thiadiazine, la thiazolidine, la thiazoline et la thiomorpholine, G étant non substitué ou substitué une, deux, trois ou quatre fois indépendantes les unes des autres par un groupe oxo ou par R5,
R1; R2, R3, R4 et R5, indépendamment les uns des autres, sont identiques ou différents et représentent
a) un atome d'hydrogène,
b) F,
c) Cl,
d) un groupe alkyle en C₁-C₄, le groupe alkyle étant non substitué ou substitué une à trois fois par R13,
e) un groupe fluoroalkyle en C₁-C₃,
f) un groupe phényle, le groupe phényle étant non substitué ou substitué une à trois fois par R13,
g) un groupe hétéroaryle en C₄-C₁₄, le groupe hétéroaryle étant choisi parmi les groupes acridinyle, azaindole (1*H*-pyrrolopyridinyle), azabenzimidazolyle, azaspirodécanyle, azépinyle, azétidinyle, benzimidazolyle, benzofuranyle, benzothiofuranyle, benzothiophényle, benzoxazolyle, benzothiazolyle, benzotriazolyle, benzotétrazolyle, benzisoxazolyle, benzisothiazolyle, carbazolyle, 4a*H*-carbazolyle, carbolinyle, chromanyle, chroményle, cinnolinyle, décahydrochinolinyle, 4,5-dihydrooxazolinyle, dioxazolyle, dioxazinyle, 1,3-dioxolanyle, 1,3-dioxolényle, 3,3-dioxo[1,3,4]oxathiazinyle, 6*H*-1,5,2-dithiazinyle, dihydrofuro[2,3-b]-tétrahydrofuranyle, furanyle, furazanyle, imidazolidinyle, imidazolinyle, imidazolyle, indanyle, 1*H*-indazolyle, indolinyle, indolizinyle, indolyle, 3*H*-indolyle, isobenzofuranyle, isochromanyle, isoindazolyle, isoindolinyle, isoindolyle, isoquinolinyle, isothiazolyle, isothiazolidinyle, isothiazolinyle, isoxazolyle, isoxazolinyle, isoxazolidinyle, 2-isoxazolinyle, cétopipérazinyle, morpholinyle, naphtyridinyle, octahydroisoquinolinyle, oxadiazolyle, 1,2,3-oxadiazolyle, 1,2,4-oxadiazolyle, 1,2,5-oxadiazolyle, 1,3,4-oxadiazolyle, 1,2-oxa-thiépanyle, 1,2-oxathiolanyle, 1,4-oxazépanyle, 1,4-oxazépinyle, 1,2-oxazinyle, 1,3-oxazinyle, 1,4-oxazinyle, oxazolidinyle, oxazolinyle, oxazolyle, oxétanyle, oxocanyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purinyle, pyranyle, pyrazinyle, pyrazolidinyle, pyrazolinyle, pyrazolyle, pyrazolo[3,4-b]pyridine, pyridazinyle, pyridooxazolyle, pyridoimidazolyle, pyridothiazolyle, pyridinyle, pyridyle, pyrimidinyle, pyrrolidinyle, pyrrolidinonyle, pyrrolinyle, 2*H-*pyrrolyle, pyrrolyle, quinazolinyle, quinolinyle, 4*H-*quinolizinyle, quinoxalinyle, quinuclidinyle, tétrahydrofuranyle, tétrahydroisoquinolinyle, tétrahydroquinolinyle, tétrahydropyranyle, tétrahydropyridinyle, tétrahydrothiophényle, tétrazinyle, tétrazolyle, 6*H*-1,2,5-thiadiazinyle, 1,2,3-thiadiazolyle, 1,2,4-thiadiazolyle, 1,2,5-thiadiazolyle, 1,3,4-thiadiazolyle, thianthrényle, 1,2-thiazinyle, 1,3-thiazinyle, 1,4-thiazinyle, 1,3-thiazolyle, thiazolyle, thiazolidinyle, thiazolinyle, thiényle, thiétanyle, thiénothiazolyle, thiénooxazolyle, thiénoimidazolyle, thiétanyle, thiomorpholinyle, thiophénolyle, thiophényle, thiopyranyle, 1,2,3-triazinyle, 1,2,4-triazinyle, 1,3,5-triazinyle, 1,2,3-triazolyle, 1,2,4-triazolyle, 1,2,5-triazolyle, 1,3,4-triazolyle, et xanthényle " et étant non substitué ou substitué une, deux, trois ou quatre fois indépendantes les unes des autres par R13,
h) un groupe cycloalkyle en C₃-C₈, ledit groupe cycloalkyle étant non substitué ou substitué une, deux, trois ou quatre fois indépendantes les unes des autres par R13,
i) un résidu cyclique à 3 à 7 chaînons qui est choisi parmi les groupes azépine, azétidine, aziridine, azirine, 1,4-diazépane, 1,2-diazépine, 1,3-diazépine, 1,4-diazépine, diaziridine, diazirine, dioxazole, dioxazine, dioxole, 1,3-dioxolène, 1,3-dioxolane, furane, imidazole, imidazoline, imidazolidine, isothiazole, isothiazolidine, isothiazoline, isoxazole, isoxazoline, isoxazolidine, 2-isoxazoline, cétomorpholine, cétopipérazine, morpholine, 1,2-oxathiépane, 1,2-oxathiolane, 1,4-oxazépane, 1,2-oxazine, 1,3-oxazine, 1,4-oxazine, oxazole, oxaziridine, oxétane, oxirane, pipérazine, pipéridine, pyrane, pyrazine, pyrazole, pyrazoline, pyrazolidine, pyridazine, pyridine, pyrimidine, pyrrole, pyrrolidine, pyrrolidinone, pyrroline, tétrahydrofurane, tétrahydropyrane, tétrahydropyridine, tétrazine, tétrazole, thiadiazine, thiadiazole, 1,2-thiazine, 1,3-thiazine, 1,4-thiazine, 1,3-thiazole, thiazole, thiazolidine, thiazoline, thiényle, thiétane, thiomorpholine, thiopyrane, 1,2,3-triazine, 1,2,4-triazine, 1,3,5-triazine, 1,2,3-triazole, et 1,2,4-triazole, et qui est non substitué ou substitué une, deux, trois ou quatre fois indépendantes les unes des autres par R13,
j) -O-CF₃,
k) un groupe -O-alkyle en C₁-C₄, le groupe alkyle étant non substitué ou substitué une à trois fois par R13,
l) un groupe -N(R10)-alkyle en C₁-C₄, le groupe alkyle étant non substitué ou substitué une à trois fois par R13,
m) -CN,
n) -OH,
o) un groupe phényloxy, le groupe phényloxy étant non substitué ou substitué une à trois fois par R13,
p) -C(O)-O-R11
q) -C(O)-N(R11)-R12,
r) -N(R11)-R12,
s) -N(R10)-SO₂-R10,
t) -S-R10,
v) -SOₙ-R10, n valant 1 ou 2,
w) -SO₂-N(R11)-R12,
x) -C (O) -R10, ou
y) l'un au moins de R1, R2, R3 et R4 est absent dans le cas où l'un ou plusieurs de J1, J2, J3 et J4 représentent un atome d'azote,
R10 représente un atome d'hydrogène, un groupe fluoroalkyle en C₁-C₃ ou un groupe alkyle en C₁-C₆,
R11 et R12, indépendamment l'un de l'autre, sont identiques ou différents et représentent
a) un atome d'hydrogène,
b) un groupe alkyle en C₁-C₄, le groupe alkyle étant non substitué ou substitué une, deux ou trois fois indépendantes les unes des autres par R13,
c) un groupe phényle, le groupe phényle étant non substitué ou substitué une, deux ou trois fois indépendantes les unes des autres par R13, ou
d) un groupe hétéroaryle en C₄-C₁₄, le groupe hétéroaryle étant tel que défini ci-dessus et étant non substitué ou substitué une, deux ou trois fois indépendantes les unes des autres par R13,
R13 représente F, Cl, un groupe -CN, =O, -OH, alkyle en C₁-C₈, alcoxy en C₁-C₈, -CF₃, phényloxy, -C(O)-R10, -C(O)-O-R17, -C(O)-N(R17)-R18, -N(R17)-R18, -N(R10)-SO₂-R10, -S-R10, -SOₙ-R10, n valant 1 ou 2, -SO₂-N(R17)-R18, phényle, le groupe phényle étant non substitué ou substitué une, deux ou trois fois indépendantes les unes des autres par R14, hétéroaryle en C₄-C₁₄, le groupe hétéroaryle étant tel que défini ci-dessus et étant non substitué ou substitué une, deux ou trois fois indépendantes les unes des autres par R14, cycloalkyle en C₃-C₆, ledit groupe cycloalkyle étant non substitué ou substitué une, deux ou trois fois indépendantes les unes des autres par R14, ou un résidu cyclique à 3 à 7 chaînons qui est tel que défini ci-dessus et qui est non substitué ou substitué une, deux ou trois fois indépendantes les unes des autres par R14,
R14 représente F, Cl, un groupe -OH, =O, -CN, -CF₃, alkyle en C₁-C₈, alcoxy en C₁-C₄, -C(O)-OH, -NH₂, -C(O)-O-alkyle en C₁-C₄, (alkyl en C₁-C₈) sulfonyle, -C(O)-NH₂, -C(O)-NH-alkyle en C₁-C₈, -C(O)-N-[alkyle en C₁-C₈]₂, -S-R10, -N(R10)-C(O)-NH-alkyle en C₁-C₈, ou -N(R10)-C(O)-N-[alkyle en C₁-C₈]₂,
R17 et R18, indépendamment l'un de l'autre, sont identiques ou différents et représentent
a) un atome d'hydrogène,
b) un groupe alkyle en C₁-C₄,
c) un groupe phényle, ou
d) un groupe hétéroaryle en C₄-C₁₄, le groupe hétéroaryle étant tel que défini ci-dessus, et X représente Cl, Br ou I.

3. Procédé selon la revendication 1 dans lequel l'un des composés de formule I suivants est préparé :
2,3-dihydro-1*H*-benzo[d]pyrrolo[1,2-a]imidazole ;
7-méthyl-2,3-dihydro-1*H*-benzo[d]pyrrolo[1,2-a]imidazole ;
6-méthyl-2,3-dihydro-1*H*-benzo[d]pyrrolo[1,2-a]imidazole ;
7-méthoxy-2,3-dihydro-1*H*-benzo[d]pyrrolo[1,2-a]imidazole ;
ester méthylique d'acide 5-méthyl-2,3-dihydro-1*H-*benzo[d]pyrrolo[1,2-a]imidazole-6-carboxylique ;
2-méthoxy-7,8-dihydro-6*H*-pyrrolo[2',1':2,3]imidazo[4,5-b]pyridine ;
2,6-diméthyl-7,8-dihydro-6*H*-pyrrolo[2',1':2,3]imidazo[4,5-b]pyridine ; 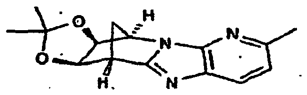
1,2,3,4-tétrahydro-benzo[4,5]imidazo[1,2-a]pyridine ;
3,9-diméthyl-6,7,8,9-tétrahydro-dipyrido[1,2-a;3',2'-d] imidazole ;
7-chloro-4,4-diphényl-1,2,3,4-tétrahydrobenzo[4,5]imidazo[1,2-a]pyridine ;
diméthyl-(S)-7,8,9,10-tétrahydro-6*H-*benzo[4,5]imidazo[1,2-a]azépin-6-yl-amine ;
3-méthyl-5,6,7,8,9,10-hexahydro-4,4b,11-triazacycloocta[a]indène ;
2-méthyl-6,7,8,9,10,11-hexahydro-5*H*-4,4b,12-triazacyclonona[a]indène ;
3-méthyl-8,8-diphényl-6,7,8,9-tétrahydro-dipyrido[1,2-a;3',2'-d]imidazole ; 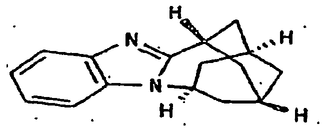
2-méthyl-8,8-diphényl-6,7,8,9-tétrahydro-dipyrido[1,2-a:3',2'-d]imidazole ;
5,6,7,8,9,10-hexahydro-1,4b,11-triaza-cyclooct[a]indène ; ou
3-méthoxy-6,7,8,9,10,11-hexahydro-5*H*-4b,12-diazacyclonon[a]indène.

4. Procédé selon la revendication 1, dans lequel le catalyseur au palladium est choisi parmi : les alcanoates de Pd, les complexes d'alcanoates de Pd, les acétonates de Pd, les halogénures de Pd, les complexes d'halogénures de Pd, et les complexes Pd-phosphine.

5. Procédé selon la revendication 4, dans lequel le catalyseur au palladium est choisi parmi : l'acétate de palladium (II), le trifluoroacétate de palladium (II), le tris(dibenzylidène-acétone)dipalladium(0), le produit d'addition du tris(dibenzylidène-acétone)dipalladium(0) et du chloroforme, le chlorure de palladium (II), le chlorure de 2,2'-bis(diphénylphosphino)-1,1'-binaphtylpalladium(II), l'acétato-(2'-di-*tert*-butylphosphino-1,1'-biphényl-2-yl)palladium (II), le (1,2-bis(diphénylphosphino)éthane)dichloropalladium (II), le bis[1,2-bis(diphénylphosphino)éthane]palladium (0), le perchlorate de [(2*S*,3*S*)-bis(diphénylphosphino)-butane][eta3-allyl]palladium (II), et le dimère de 1,3-bis(2,4,6-triméthylphényl)-imidazol-2-ylidène(1,4-naphtoquinone)palladium (0).

6. Procédé selon la revendication 1, dans lequel le catalyseur au cuivre est choisi parmi : les sels halogénés de cuivre (I), et les oxydes de cuivre.

7. Procédé selon la revendication 6, dans lequel le catalyseur au cuivre est choisi parmi : le chlorure de cuivre (I), le bromure de cuivre (I), l'iodure de cuivre (I), et l'oxyde de cuivre (I).

8. Procédé selon les revendications 1 à 7, dans lequel la base est choisie dans le groupe des carbonates, phosphates, fluorures, alcoxydes et hydroxydes avec un métal approprié comme contre-ion.

9. Procédé selon la revendication 8, dans lequel la base est choisie dans le groupe suivant : carbonate de potassium, phosphate de potassium, et carbonate de césium.

10. Procédé selon les revendications 1 à 9, dans lequel le solvant aprotique est choisi dans le groupe suivant : benzène, toluène, xylène, mésitylène, acétonitrile, tétrahydrofurane, diméthylformamide, n-méthylpyrrolodinone, diméthylacétamide, diméthylsulfoxyde, éther 2-méthoxyéthylique, et pyridine.

11. Procédé selon les revendications 1 à 10, dans lequel la réaction entre les composés de formule II et de formule III est réalisée dans une gamme de température de 60 °C à 150 °C, de préférence de 70 °C à 90 °C.

12. Procédé selon les revendications 1 à 11, dans lequel le deuxième solvant est choisi dans le groupe suivant : méthanol, éthanol, propanol, acide acétique, chlorure de méthylène, diméthylformamide, tétrahydrofurane, pyridine, *p*-xylène, acétate d'éthyle, benzène, toluène, xylène, mésitylène, et acétonitrile.

13. Procédé selon la revendication 12, dans lequel le deuxième solvant est choisi dans le groupe suivant : méthanol, éthanol, acide acétique, chlorure de méthylène, diméthylformamide, pyridine, et p-xylène.

14. Procédé selon les revendications 1 à 13, dans lequel le réactif réducteur est choisi dans le groupe suivant : H₂/Ni de Raney, H₂/Pd-C, H₂/PtO₂, H₂/Ru, NaBH₄/NiCl₂, NaBH₄/FeCl₂, H₃PO₂/Pd-C, Sn/HCl, SnCl₂/HCl, Fe/HOAc, Fe/HCl, FeSO₄/HCl, Fe/FeSO₄, Zn/HCl, Na₂S, et Na₂S₂O₄.
